(19) **Europäisches Patentamt / European Patent Office / Office européen des brevets**

(11) **EP 4 069 324 B1**

(12) # EUROPEAN PATENT SPECIFICATION

(45) Date of publication and mention
of the grant of the patent:
**21.05.2025  Bulletin 2025/21**

(21) Application number: **20897608.4**

(22) Date of filing: **01.12.2020**

(51) International Patent Classification (IPC):
*A61L 24/10* (2006.01)    *A61L 24/08* (2006.01)
*A61L 24/02* (2006.01)    *A61K 9/16* (2006.01)
*A61K 9/00* (2006.01)    *A61K 38/38* (2006.01)
*A61K 47/06* (2006.01)    *A61P 7/04* (2006.01)
*A61K 9/10* (2006.01)    *A61K 38/36* (2006.01)
*A61K 38/39* (2006.01)    *A61K 38/48* (2006.01)
*A61L 24/04* (2006.01)

(52) Cooperative Patent Classification (CPC):
(C-Sets available)
**A61L 24/106; A61K 9/0014; A61K 9/10;
A61K 9/1658; A61K 38/363; A61K 38/39;
A61K 38/4833; A61L 24/043; A61P 7/04;**
A61L 2400/04                              (Cont.)

(86) International application number:
**PCT/CN2020/133059**

(87) International publication number:
**WO 2021/109979 (10.06.2021 Gazette 2021/23)**

(54) **FLOWABLE FIBRINOGEN THROMBIN PASTE**

FLIESSFÄHIGE FIBRINOGENTHROMBINPASTE

PÂTE DE THROMBINE FIBRINOGÈNE FLUIDE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority:  **06.12.2019  PCT/CN2019/123652**

(43) Date of publication of application:
**12.10.2022  Bulletin 2022/41**

(73) Proprietor: **Guangzhou Bioseal Biotech Co., Ltd.
Guangzhou, Guangdong 510530 (CN)**

(72) Inventors:
• **TIAN, Yuan**
**Guangzhou Science City, Guangdong 510530
(CN)**
• **LI, Hai**
**Guangzhou Science City, Guangdong 510530
(CN)**
• **FENG, Dengmin**
**Guangzhou Science City, Guangdong 510530
(CN)**

• **TENG, Ling**
**Guangzhou Science City, Guangdong 510530
(CN)**
• **WANG, Yalin**
**Guangzhou Science City, Guangdong 510530
(CN)**
• **CAI, Yinghua**
**Guangzhou Science City, Guangdong 510530
(CN)**
• **WANG, Peiqiu**
**Guangzhou Science City, Guangdong 510530
(CN)**
• **XIE, Tingwan**
**Guangzhou Science City, Guangdong 510530
(CN)**

(74) Representative: **Carpmaels & Ransford LLP
One Southampton Row
London WC1B 5HA (GB)**

(56) References cited:
WO-A1-2013/079604     WO-A1-2013/082073
WO-A1-2015/086028     WO-A1-2018/033835

EP 4 069 324 B1

**(Cont. next page)**

WO-A1-2019/160717    WO-A1-98/16250
WO-A2-2007/110694    WO-A2-2013/079605

(52) Cooperative Patent Classification (CPC): (Cont.)

C-Sets
A61K 38/363, A61K 2300/00;
A61K 38/39, A61K 2300/00;
A61K 38/4833, A61K 2300/00;
A61L 24/043, C08L 89/06

**Description**

FIELD OF THE INVENTION

The present invention relates, *inter alia,* to hemostatic compositions comprising a blend of fibrinogen, thrombin, and a hydrophobic dispersant.

BACKGROUND OF THE INVENTION

The control of bleeding is essential and critical, especially in surgical procedures, to minimize blood loss, to reduce post-surgical complications, and to shorten the duration of the surgery in the operating room.

Dry sealant powder, when mixed with fluid, can form a paste or slurry that is useful as a flowable, extrudable and injectable hemostat is made of plasma-derived components: e.g., (a) fibrinogen mixture, primarily composed of fibrinogen optionally along with catalytic amounts of Factor XIII and (b) a high potency thrombin. The main disadvantage of this approach is the time needed to mix the powder with the liquid, knead it into a paste and backfill it into the delivery device of choice, all at the time of need and at the point of use. The manipulations are all time consuming and potentially might compromise the sterility of the delivered product.

US Patent No. 4965203 entitled: "Purified thrombin preparations" discloses improved thrombin formulations, stable at room temperature using stabilizing quantities of a polyol and a buffer at specific pHs.

European Patent No. 1140235 entitled: "Fibrin-based glue granulate and corresponding production method" relates to a flowable fibrin glue granulate containing thrombin, Factor XIII, fibrinogen and a calcium salt in the form of granules with a particle size of more than 50 μm to 1000 μm.

US Patent No. 9717821 entitled: "Formulations for wound therapy" relates to formulations comprising a dry powder fibrin sealant comprised of a mixture of fibrinogen and/or thrombin, for use in the treatment of wounds or injuries, in particular for use as a topical hemostatic composition or for surgical intervention.

US Patent No. 7109163 entitled: "Hemostatic compositions and devices" relates to sterilized and unsterilized hemostatic compositions that contain a biocompatible liquid having particles of a biocompatible polymer suitable for use in hemostasis and which is substantially insoluble in the liquid, up to about 20 percent by weight of glycerol and about 1 percent by weight of benzalkonium chloride, each based on the weight of the liquid, all of which are substantially homogenously dispersed throughout the liquid to form a substantially homogenous composition.

Retzinger et al. have shown that fibrinogen adsorbs spontaneously from aqueous media containing that protein to droplets of liquid hydrophobic phases dispersed in those same media *(*Arterioscler Thromb. Vasc. Biol. 1998, 1948-1957).

WO 2013/082073 describes hemostatic pads comprising a bioabsorbable scaffolding material, a lyophilized thrombin powder, a lyophilized fibrinogen powder, and a meltable binder powder, with all powders disposed on the bioabsorbable scaffolding material.

WO 2013/079604 describes a method for manufacturing a drug-delivery composition which includes providing at least a pharmaceutically active composition, providing a hydrophobic matrix, and mixing the hydrophobic matrix and the pharmaceutically active composition to form a paste-like or semi-solid drug-delivery composition.

WO 98/16250 describes stable non-aqueous formulations which are suspensions of proteinaceous substances or nucleic acids in non-aqueous, anhydrous, aprotic, hydrophobic, non-polar vehicles with low reactivity.

WO 2019/160717 describes hemostatic compositions comprising at least partially integrated agglomerated ORC fibers, fibrinogen, and thrombin.

WO 2015/086028 describes dry compositions comprising one or more polyols which, upon addition of an aqueous medium, form substantially homogenous pastes suitable for use in hemostasis procedures.

WO 2018/033835 describes hemostatic compositions comprising at least partially integrated agglomerated ORC fibers, fibrinogen, and thrombin.

WO 2013/079605 describes a method for manufacturing drug-delivery compositions, which includes providing at least one pharmaceutically active compound, a dry powder comprising at least a polymer, and an aqueous solution. The dry powder, the pharmaceutically active compound and the aqueous solution are mixed to form a paste-like or semi-solid drug-delivery composition, wherein the aqueous solution is added in an amount of less than or equal to twice the total dry mass of the dry powder.

WO 2007/110694 describes biodegradable carriers synthesized from ricinoleic acid oligoesters and aliphatic molecules having at least one carboxylic acid and at least one hydroxy or carboxylic acid group that are liquids or pastes at temperatures below 37 °C.

## SUMMARY OF THE INVENTION

The scope of the invention is defined by the claims. Any references in the description to methods of treatment refer to the products of the present invention for use in a method for treatment.

The present invention relates, *inter alia,* to hemostatic compositions (also referred to as "compositions") comprising a blend of fibrinogen, thrombin, and a hydrophobic dispersant.

An object of the present invention is to provide a composition for preparing a pasty spreadable hemostat having a therapeutic or medicinal value, which may easily be applied to a site of need, including in difficult-to-reach areas of the body.

Fibrinogen interacts with thrombin in aqueous media. Thus, for a stable liquid/flowable composition comprising both fibrinogen and thrombin, non-aqueous medium (dispersant) should be used prior to application on bleeding sites e.g., for sealing and hemostasis.

Finding a suitable formulation having an appropriate consistency is not straightforward, since the commonly used dispersant, such as water or hydrophilic dispersant, cannot be used without compromising the pastiness, adhesiveness, and/or hemostatic functions.

The present inventors have found that hydrophobic liquids may provide fluidity of fibrinogen and thrombin particles and, at the same time maintain their function once applied in/on a bleeding tissue.

Furthermore, as the hemostatic action is time-critical in order to meet medical needs, the disclosed composition can be used immediately, without reconstitution, thereby preventing the hassle and saving time for the practitioner as well as minimizing a risk of contamination.

As such, the disclosed composition has been demonstrated to be an easy-to-use, stable and efficacious topical hemostat.

The hemostatic compositions can be used in a paste form as a hemostat for various surgical and wound healing topical applications, such as anti-adhesion barriers, hemostats, tissue sealants, etc.

According to an aspect of the present disclosure, there is provided a composition comprising: (i) a blend of fibrinogen and thrombin, wherein the blend is in the form of a plurality of particles, and (ii) a hydrophobic dispersant, wherein the composition is in the form of a paste at at-least one temperature in the range of 10 °C to 37 °C.

In some embodiments, the respective weight ratio of said blend to the hydrophobic dispersant ranges from 0.5:1 to below 0.9:1.

In some embodiments of any aspect of the present disclosure, the composition or the paste further comprises collagen. In some embodiments, the collagen is present at a concentration of below 20 %, by respective weight. In some embodiments, the collagen is present at a concentration of at least about 10 %, by weight.

In some embodiments, the paste is characterized by a yield point ranging from about 0.1 to about 15 Pa, as measured at a temperature of 25 °C and frequency of 10 rad/sec.

In some embodiments of any aspect of the present disclosure, the hydrophobic dispersant is selected from vegetable oil, mineral oil, and a combination thereof.

In some embodiments of any aspect of the present disclosure, the hydrophobic dispersant is selected from oil comprising one or more from: soybean oil, olive oil, cholesteryl oleate, corn oil, triolein, safflower oil, squalene, squalane, and dodecane, and any mixture thereof.

In some embodiments of any aspect of the present disclosure, the hydrophobic dispersant comprises mineral oil.

In some embodiments of any aspect of the present disclosure, the fibrinogen is present at a concentration of 60 % to 95 %, or 70 % to 90 %, by weight of the blend.

In some embodiments of any aspect of the present disclosure, the fibrinogen is present at a concentration of 60 % to 85 %, by weight of the blend, and the thrombin is present at a concentration of 5 %, to 10 %, by weight of the blend.

In some embodiments of any aspect of the present disclosure, the fibrinogen and thrombin are present in the blend at a respective weight ratio ranging from 5:1 to 20:1, respectively, e.g., 5:1, 6:1, 7:1, 8:1, 9:1, 10:1, 11:1, 12:1, 13:1, 14:1, 15:1, 16:1, 17:1, 18:1, 19:1, or 20:1, respectively, by weight, including any range therebetween.

In some embodiments of any aspect of the present disclosure, the blend comprises less than 6 % water, by weight.

In some embodiments of any aspect of the present disclosure, the composition comprises less than 5 % water, less than 4.5 % water, less than 4 % water, less than 3.5 % water, or less than 3 % water, by total weight. In some embodiments of any aspect of the present disclosure, the composition comprises water at a concentration of up to 5 % water, up to 4.5 % water, up to 4 % water, up to 3.5 % water, or up to 3 % water, by total weight.

In some embodiments of any aspect of the present disclosure, the blend further comprises one or more excipients selected from: amino acids, albumin, saccharides, and any derivative or mixture thereof. The term "derivative" refers to compound containing essential elements of the parent substance.

In some embodiments of any aspect of the present disclosure, the excipient comprises a calcium salt and/or lysine.

In some embodiments of any aspect of the present disclosure, the plurality of particles is characterized by a median particle size of up to about 270 $\mu$m.

The term "median" in the context of particles size refers to the particle size that divides the population in half such that 50 % of the population is greater than or less than this size.

In some embodiments of any aspect of the present disclosure, the plurality of particles is characterized by a median particle size of about 10 to about 270 $\mu$m.

In some embodiments of any aspect of the present disclosure, the composition comprises oxidized cellulose (OC) at a concentration of less than 12 %, or, in some embodiments is even devoid of OC.

In some embodiments of any aspect of the present disclosure, the blend and/or the composition are devoid of OC.

In some embodiments of any aspect of the present disclosure, the OC comprises oxidized regenerated cellulose (ORC).

In some embodiments of any aspect of the present disclosure, the blend is spray-dried mixed or alternatively, or additionally, is high-shear mixed. In some embodiments of any aspect of the present disclosure, the blend is high-shear mixed.

In some embodiments of any aspect of the present disclosure, the thrombin originates from porcine plasma.

In some embodiments of any aspect of the present disclosure, the composition is a hemostatic composition.

In some embodiments of any aspect of the present disclosure, the composition is for use in a method for treating a bleeding tissue.

In some embodiments of any aspect of the present disclosure, the composition comprises: (i) a blend of fibrinogen, thrombin, wherein the blend is in the form of a plurality of particles, and (ii) a hydrophobic dispersant comprising mineral oil, and optionally (iii) one or more excipients or additives selected from calcium salt and/or lysine, wherein: the ratio (also referred to as "oil ratio") of the blend (powder) to the hydrophobic dispersant ("medium") ranges from 0.5:1 to 0.9 (w/w); the plurality of particles is characterized by a median particle size of about 100 to about 270 $\mu$m, and wherein the composition is in the form of a paste at at-least one temperature in the range of 10 °C to 37 °C.

The composition according to the invention can be used in a method for preparing a fibrin adhesive sealant in/on an injured tissue (referred to as: "adhesive sealant preparation method"), by applying the disclosed composition in any aspect or embodiments thereof, in/on a surface of the tissue.

In some instances, the tissue is a soft tissue. In some embodiments, there is provided a sealant layer obtained by the use of the composition (i.e. by the "adhesive sealant preparation method") in any embodiments thereof. In some embodiments, the obtained sealant layer is characterized by minimum bond strength of at least 3.5 N/m, as measured by the T-peel test (ASTM F2256-052015).

According to another aspect of the present disclosure, there is provided a composition comprising: (i) a blend of fibrinogen, thrombin, wherein the blend is in the form of a plurality of particles, and (ii) a hydrophobic dispersant comprising mineral oil; and optionally (iii) one or more excipients or additives or selected from calcium salt and/or lysine, wherein: the ratio of said blend to the hydrophobic dispersant ranges from 0.5 to below 0.9 (w/w); the one or more excipients or additives are selected from calcium salt and lysine; the plurality of particles is characterized by a median particle size of about 10 to about 100 $\mu$m, and wherein the composition is in the form of a paste at at-least one temperature in the range of 10 °C to 37 °C, and optionally is devoid of OC (e.g., ORC).

According to another aspect of the present disclosure, there is provided a kit comprising:

    a. a container containing the disclosed composition in any aspect and/or embodiment thereof;
    b. an applicator for applying the composition to a tissue, and optionally,
    c. instructions for use.

Unless otherwise defined, all technical and/or scientific terms used herein have the same meaning as commonly understood by one of ordinary skill in the art to which the invention pertains. Although methods and materials similar or equivalent to those described herein can be used in the practice or testing of embodiments of the invention, exemplary methods and/or materials are described below. In case of conflict, the patent specification, including definitions, will control.

BRIEF DESCRIPTION OF THE DRAWINGS

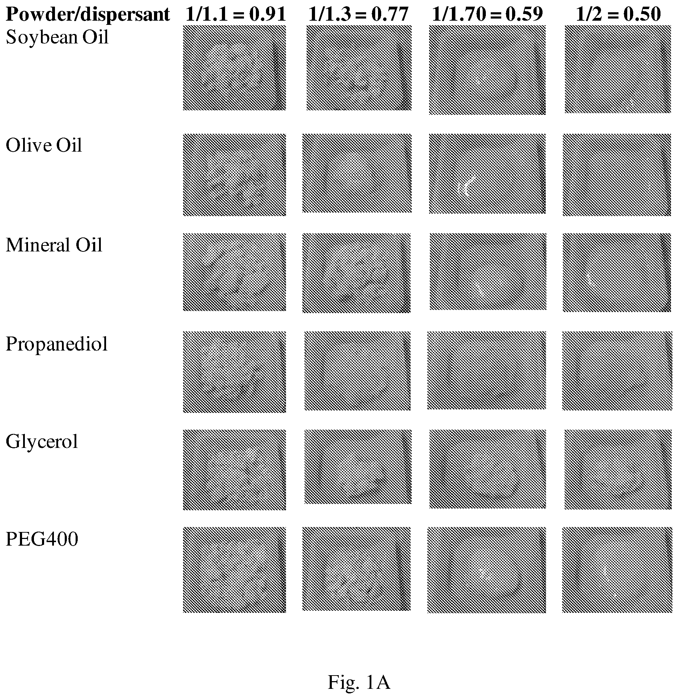

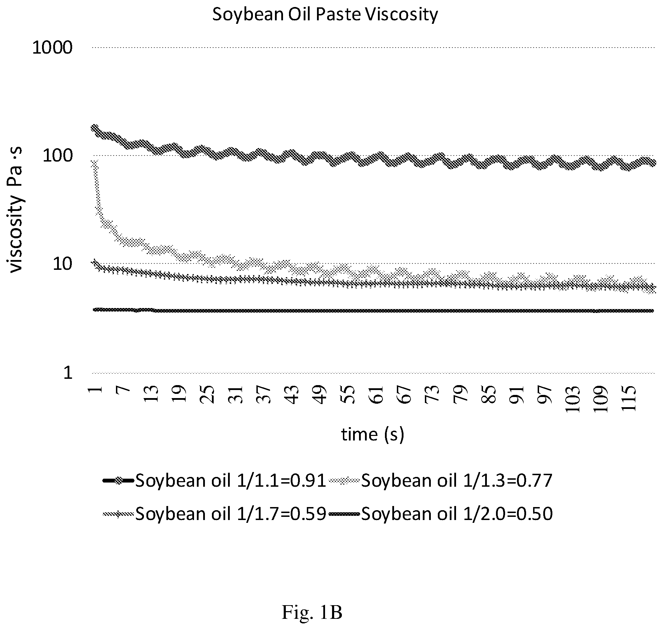

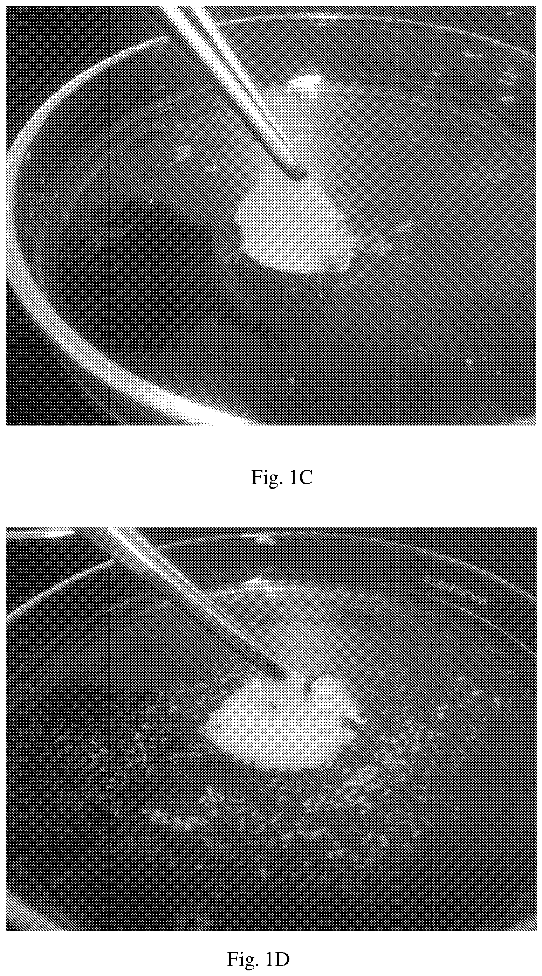

**Figs. 1A-D** demonstrate the flowability of selected samples as described in Example 1, showing: photographic images demonstrating the flowability of selected samples (Fig. 1A; from left panels to the right: powder/dispersant weight ratios: 1/1.1= 0.91, 1/1.3 = 0.77, 1/1.70 = 0.59, 1/2 = 0.50; from the upper panels to the lower: soybean oil, olive oil, mineral oil, propanediol, glycerol, polyethylene glycol (PEG) 400); a graph showing the viscosity behavior of compositions with several powder to soybean oil ratios (Fig. 1B); and comparative photographic images showing that when hydrophobic dispersant, such as soybean oil, is used the paste forms an intact layer after 7 min curing with $H_2O$ (Fig. 1C), whereas when the dispersant is PEG 400, the paste fails to form an intact layer under the same conditions (Fig. 1D).

**Fig. 2** presents a bar graph illustrating the average gelation time of each tested sample, 1-8, according to Table 2 below (N=3).

**Figs. 3A-B** present graphs demonstrating the main affecting parameters (Fig. 3A) and interactions (Fig. 3B) for gelation time according to the statistical analysis shown in Table 3 ("siz" denotes size, "percenta" denotes percentage).

**Figs. 4A-B** present: a graph showing the means of average load per width for various paste formulations samples according to Table 4 below (Fig. 4A; the pooled standard deviation was used to calculate the intervals); and Tukey's simultaneous tests (Fig. 4B) presenting the different of means for each pair of compositions (if an interval does not contain "zero", the corresponding means are significantly different).

**Fig. 5** presents a bar graph illustrating the load per width ("T-peel test") for each tested sample according to Table 7 below.

**Figs. 6A-B** present graphs demonstrating the main affecting parameters (Fig. 6A) and interactions (Fig. 6B) for T-peel test according to the statistical analysis shown in Table 12 below ("siz" denotes size, "percenta" denotes percentage).

**Fig. 7** presents a bar graph showing formulation homogeneity evaluated by liquid/solid phase separation (remnant) within applicator after a fixed pressing force of 80N was applied. The results suggest that smaller particles may achieve better homogeneity paste for formulation samples presented Table 13 below.

**Fig. 8** presents a bar graph showing stability evaluation: remnant was evaluated on samples described in Table 14 below (left quartet: particles size of 10-52 $\mu$m from Group A; right quartet: particles size of 23-83 $\mu$m from Group B) at 4 time points: 0, 7, 23, and 30 days at room temperature formulation homogeneity was evaluated by liquid/solid phase separation (remnant) within applicator after a fixed pressing force of 80N had been applied.

**Fig. 9** presents graphs showing the results of three-interval thixotropy test, carried out on Samples (1) to (5) (marked by "1" to "5" in the graphs' area) described in Example 5 in the Examples section. The dotted lines delimit time intervals "I", "II", and "III".

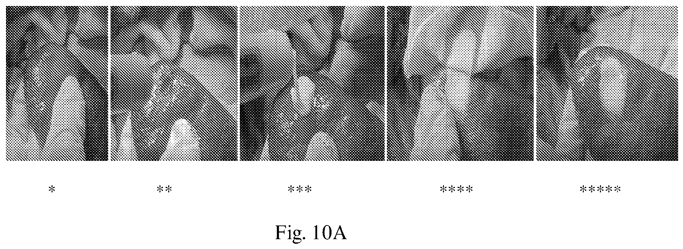

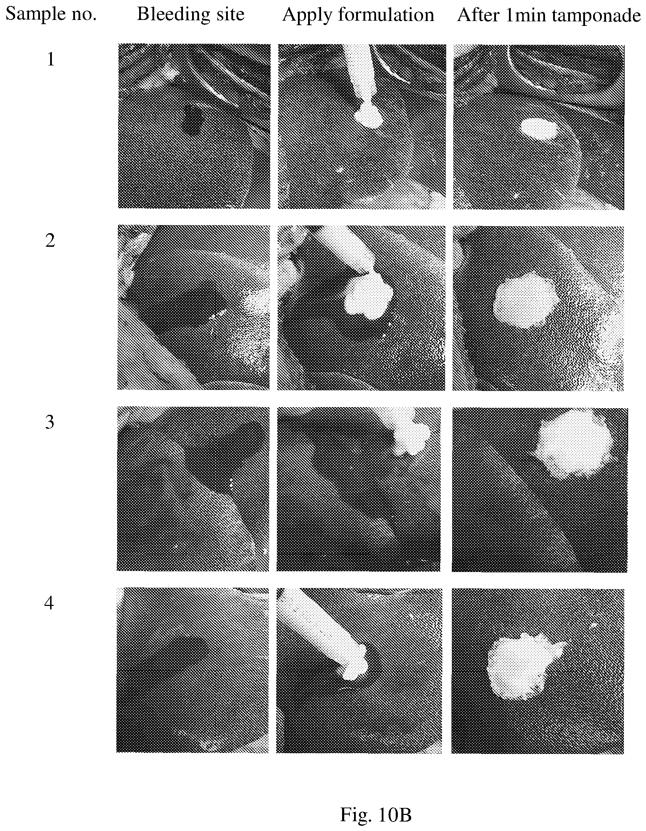

**Figs. 10A-B** present photographic images outlining an *in vivo* application carried out on porcine spleen, with a 6 mm biopsy puncher of an exemplary composition disclosed herein using spleen biopsy punch model under heparinized condition: a visual flow chart images (Fig. 10A; from left panel to right: *the bleeding site in the injured tissue; **approaching the syringe pre-filled with the composition in a location close to the bleeding site; ***applying the composition; ****tamponade for 1 min; and *****the sealant in/on an injured tissue; and photographic images of tested samples 1-4, respectively, from the upper to the lower panels) as presented in Table 15 for *in vivo* application (from left to right panels: the bleeding site, applying the composition, and after 1 min tamponade), showing that there is no significant effect of the particles size on the hemostatic efficacy (Fig. 10B).

<u>DESCRIPTION OF EMBODIMENTS OF THE INVENTION</u>

Commercial preparations of thrombin and fibrinogen blend exist as a powder. Application of powder for a limited and specific site can be difficult to achieve. For this purpose, it is advantageously to use a flowable composition comprising the powder.

Fibrinogen and thrombin can be kept unreacted in anhydrous hydrophilic dispersant and once applied into the bleeding site, the anhydrous hydrophilic dispersant attracts water facilitating fibrinogen and thrombin reaction and consequently forming a clot.

However, in the present disclosure it has been surprisingly found that using hydrophobic dispersants provides significant advantages over anhydrous hydrophilic dispersants.

According to an aspect of the present disclosure, there is provided a composition comprising a blend of fibrinogen and thrombin dispersed in a hydrophobic dispersant in the form of a paste. Advantageously, the paste can be flowable and/or homogenous. Ranges of blend to dispersant of e.g., from 0.3:1, 0.4:1, or 0.5:1 to below 0.9:1 (w/w, respectively) were found by the present investors to be suitable. In some embodiments, the composition is in the form of a paste at around room temperature.

By "around the room temperature" it is meant to refer to at least one temperature value within the range of 10 to 40 °C, 10 to 37 °C, or 15 to 37 °C, e.g., 10, 15, 20, 25, 30, 35, 37, or 40°C, including any value and range therebetween.

By "0.3:1 to below 0.9:1 (w/w)" it is meant, for example, 0.3:1, 0.35:1, 0.4:1, 0.45:1, 0.5:1, 0.55:1, 0.6:1, 0.65:1, 0.7:1, 0.75:1, 0.8:1, or approaching 0.9:1 w/w. In some embodiments, the ratio of the blend to the hydrophobic dispersant is from about 0.6:1 to about 0.8:1, e.g., about 0.8:1 (w/w). In some embodiments, the ratio of the blend to the hydrophobic dispersant is about 0.7:1 (w/w).

**Figure 1A,** discussed in Example 1 below, shows homogenous pastes at respective ratios of powder to hydrophobic dispersant of 0.50:1, 0.59:1, 0.71:1, and 0.83:1 at room temperature. In contrast, compositions made with similar ratios of the powder to hydrophilic dispersant (except from polyethylene glycol; PEG400), failed to exhibit homogeneous paste at these temperatures. Also, the results show that adhesive properties of a paste prepared with PEG400 were inferior as compared to paste prepared with hydrophobic dispersants at similar ratios of powder to dispersant. According to the instant results, paste prepared from blends comprising fibrinogen and thrombin dispersed in hydrophobic dispersants seems superior to paste prepared from blends comprising fibrinogen and thrombin dispersed with non-hydrous hydrophilic dispersants in terms to flowability, gelation, and adhesiveness, of the paste.

The results also show that mineral oil ("MO") provides the highest adhesive property compared to other paste formulations. It has been found according to the disclosure, that paste comprising larger particles (106-250 $\mu$m) exhibited quicker gelation compared to that of smaller ones (<106 $\mu$m). However, paste with larger granules typically exhibited poor homogeneity, perhaps since the paste formulation comprises two separated phases: solid particles and liquid hydrophobic media. Without being bound by any mechanism, smaller granules may mitigate the separation of phases thus improving homogeneity and the final outcome, improved adhesion. The experimental results have shown a positive effect of ORC on gelling speed or rate, however, the results of adhesion may be superior in the absence of ORC. Paste with higher oil dosage show slower gelation performance. In terms of T-peel test which reflects paste adhesion, no main effect was observed for oil dosage.

As used herein the term "formulation" refers to a vehicle composition in the form of emulsion, lotion, cream, gel, paste, etc.

It has been also found that related composition would lose efficacy, e.g., in decrease in gelation time, at a ratio of blend (powder) to the hydrophobic dispersant of below about 0.5:1 w/w.

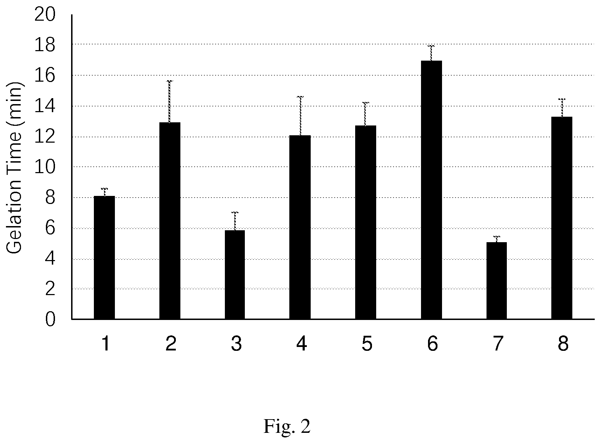

Reference is made to **Figure 2** (test paste samples "3" and "4" therein) showing that for a composition having certain set of components and properties of components at the powder-to-oil ratio of 1/1.4 (0.71:1) the gelation time is improved by about 100 %, compared to the ratio of e.g., 1/1.7 (0.58:1), which resulted in much higher gelation time.

As used herein, and unless stated otherwise, the terms "by weight", "w/w", "weight percent", or "wt. %", which may be used herein interchangeably describe the concentration of a particular substance e.g., blend out of the total weight of the corresponding mixture, solution, formulation or composition.

When referring to a "blend" it is to be understood as any form of a mixture, homogenous and non-homogenous mixture of at least the two ingredients. The blend may include other ingredients as is further detailed below. Thus, the terms "blend of" and "blend comprising" as used hereinthroughout are interchangeable.

In some embodiments, the blend is biocompatible.

In some embodiments, the term "biocompatible", or any grammatical inflection thereof, is defined as the ability of a material to perform with an appropriate host response in a specific application. More specifically, in the context of present disclosure, "biocompatibility" refers to the ability to perform as a hemostatic agent that does not elicit any undesirable effects.

Thus, a biocompatible composition typically provides a suitable environment for biological activity, without inducing any undesirable local or systemic responses in the host.

In some embodiments, the blend is in the form of a plurality of particles, typically solid particles. Additionally or alternatively, the blend may be in the form of a powder.

Herein the blend comprising fibrinogen and thrombin powder is also referred to as "FTP" or "FTP blend".

As used herein, the term "powder" refers to dispersed dry solid particles, typically in the form of a plurality of particles of a solid characterized by small size, typically, within the range of from 0.1 to 1000 micrometers.

In some embodiments, the blend is in the form of aggregate(s) or granulate(s). The term "aggregate" describes a particle formed from assembled components. Aggregates may optionally be made by a step of humidifying the powder composition; compacting, e.g., by roller and/or slugging the powder to form aggregates; dehumidifying; milling; sieving the aggregates; and optionally dosing the resulting aggregates into a storage container or into a delivery device. The term "granulate" or "granulate material" may particularly denote a conglomeration of discrete solid particles typically below 250 micron, below 110 micron, below 60 micron, or even below 20 micron. In exemplary embodiments, high shear mix is utilized to assemble particles of fibrinogen, thrombin, and optionally CaCl$_2$, and ORC into aggregates. In another embodiment, the composition is in the form of a mixture. The mixture may comprise, in exemplary embodiments, spray-dried first microparticles that comprise fibrinogen, combined with spray-dried second microparticles that comprise thrombin, e.g., human thrombin, together forming particles of e.g., 10 micron to about or above 250 micron.

The term "solid" characterizes the state of the compound or composition at room temperature (e.g., 25 °C) and at atmospheric pressure (760 mmHg), i.e. a compound or a composition of high consistency which retains its form during storage. This term in the present application also relates to non-fluid particles, or dissolved substance. As opposed to "liquid" compounds and compositions, the solid does not flow under its own weight.

The term "paste" as used herein, relates to the consistency of the composition at at-least one temperature around the room temperature, and defines a fluid mixture of solid particles. Typically, paste has no fixed shape, and is therefore not a solid or a gas. The term "paste" according to the present disclosure may also include slurry, salve, and ointment. Slurry may functionally be regarded as a thin, oily paste. A paste according to the present disclosure may also include pores comprising of an expandable gas, such as air. Accordingly, the composition is a paste, or is in pasty consistency at at-least one temperature in the range of 10 °C to 37 °C.

As used herein, the term "flowable", or any grammatical deflection thereof, in the context of paste relates to a having fluid consistency at around the room temperature and typically is capable of being administered through a syringe and/or may still flow after application of the composition in/on the bleeding site for at least 1 to 10 sec.

The paste may have a viscosity and potency which is high enough to permit its hemostatic effective use by a surgeon by dipping of a gloved finger into the paste and placing the paste over the bleeding site.

The term "flowable" also encompasses a viscous solution. The term "viscous solution" refers to a solution that has an increased resistance to flow, yet is capable of flowing, typically, but not exclusively, having a static viscosity greater than about 5 and is less than about 150 Pa·s. In some embodiments, the paste is characterized by a static viscosity of 5 to 90, 10 to 90, 10 to 90, or 20 to 80, e.g., 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, or 90 Pa·s, including any value therebetween.

The term "static viscosity" or "viscosity at the static stage" is used herein to refer to the viscosity of the composition prior to injection, typically within at up to 15 to 20 sec upon contacting the blend with the hydrophobic medium at ambient pressure and temperature conditions.

In some embodiments, the paste is characterized by viscosity of 5 to 20, e.g., 5, 6, 7, 8, 9, 10, 11, 12, 13, 14, or 15 Pa·s, including any value therebetween, within 20 to 30 sec upon contacting the blend with the hydrophobic medium at ambient pressure and temperature conditions (referred to as "ambient conditions", i.e. around room temperature and atmospheric pressure).

Reference is made to **Figure 1B** presenting a viscosity study of different powder to oil ratio, (for soybean oil model). The viscosity of the samples of the blend-to oil of 0.59 to 0.77 ratios approaches 10 Pa·s at ambient conditions. At the static stage (point 0), an appropriate static viscosity is between 10-100 Pa·s at ambient conditions, the higher the viscosity at static stage, the more stable the paste is. The rapid dropping down of the curve with the 0.77 ratio means that the paste could be squeezed out of the syringe by an acceptable force (by hand).

The terms "hydrophobic liquid", "hydrophobic solvent", "hydrophobic dispersant", "hydrophobic medium", "oil", "oily liquid", "oily medium", "non-polar solvent", "non-polar liquid", or "hydrophobic liquid" (may also be referred to as a "lipophilic liquid"), which may be used herein interchangeably, is a substance which is liquid at around room temperature and which is typically not dissolvable, or has a limited solubility in aqueous solution and is dissolvable in non-polar organic solvents.

The term "aqueous solution" refers to a solution with water as the solvent.

The terms "liquid medium", "medium", or "dispersant" may be used hereinthroughout interchangeably. Herein, the term "dispersant" is meant to refer to a liquid medium, optionally a liquid medium having a viscosity having, or is capable of providing a pasty consistency. Thus, the term "dispersant" is used in a generic meaning, and may be employed rather than the word "solvent" only to clarify that the blend comprised of fibrinogen and thrombin is substantially not soluble in such a liquid medium, but rather forms a solid-in-liquid system.

Oily liquids have oily constitution and include, for example, natural and synthetically prepared oils such as olive oil, other plant and animal-derived oils, and inorganic oils such as silicon oil and/or other mineral oils.

Non-limiting types of hydrophobic liquids include organic substances such as alkanes, particularly long-chain alkanes, cycloalkanes, including bicyclic compounds, aryls (both substituted and unsubstituted), and fatty acids.

Further non-limiting types of hydrophobic liquids include cholesteryl myristate, cholesteryl laurate, cholesteryl dodeconate, cholesteryl palmitate, cholesteryl arachidonate, cholesteryl behenate, cholesteryl linoleate, cholesteryl linolenate, cholesteryl oleate, cholesteryl stearate, olive oil, corn oil, triolein, triolein/cholesteryl oleate mixtures, safflower oil, squalene, squalane, dodecane, and any mixture thereof, e.g., olive oil and cholesteryl oleate, and triolein/cholesteryl oleate mixtures.

Hydrophobic substances can also be determined by the partition coefficient thereof.

A partition coefficient is the ratio of concentrations of a compound in the two phases of a mixture of two immiscible liquids at equilibrium. Normally, one of the solvents chosen is water while the second is hydrophobic such as octanol. The logarithm of the ratio of the concentrations of the un-ionized solute in the solvents is called "logP".

Typically, hydrophobic liquids are characterized by LogP higher than 1; hydrophilic liquids are characterized by LogP lower than 1 and amphiphilic liquids are characterized by LogP of about 1 (e.g., 0.8 to 1.2).

Typically, but not exclusively, by "limited solubility in aqueous solution" it is meant that less than 20 weight percent, less than 10 weight percent, less than 5 weight percent, or, at times, less than 1 weight percent, of the hydrophobic substance is soluble in water at about 25 °C.

As used hereinthroughout, the terms "hydrophilic", or "polar" refer to a solvent that may attract to water, dissolves in water and whose interaction with water is thermodynamically favorable. Typically, but not exclusively, hydrophilic solvents have a solubility of at least 10, 20, 30, 40, 50, 60, 70, 80, 90, 100, 200, 300, 400, 500, or at least 1000 mg/ml in water or other polar solvents at about 25 °C. Each possibility represents a separate embodiment of the present invention.

In some embodiments, the blend is dry. By "dry", in the context of blend, it is meant to refer to a blend comprising less than 8 %, less than 5 %, or less than 1 %, of water, by weight of the blend.

In some embodiments, the composition is dry, e.g., comprising less than 8 %, less than 5 %, or less than 1 %, of water, by weight of the composition, prior to its application into/onto an aqueous environment (e.g., into/onto aqueous solution or a bleeding site).

In some embodiments, the dispersant (e.g., the hydrophobic dispersant) is dry (also referred to as: "anhydrous"), e.g., comprising less than 8 %, less than 5 %, or less than 1 %, of water, by weight of the composition, prior to its application into/onto an aqueous solution, such as water, saline, or a bleeding site.

In some embodiments, the hydrophobic dispersant comprises mineral oil. The term "mineral oil" refers to one or a mixture of liquid hydrocarbons, optionally obtained from petrolatum *via* a distillation technique. The term is synonymous with "liquefied paraffin", e.g., having a CAS no.8020-83-5, "liquid petrolatum" and "white mineral oil." The term is also intended to include "light mineral oil" i.e. oil which is similarly obtained by distillation of petrolatum, but which has a slightly lower specific gravity than white mineral oil. See, e.g., Remington's Pharmaceutical Sciences, 18th Edition (Easton, Pa.: Mack Publishing Company, 1990, at pages 788 and 1323). Mineral oil may be obtained from various commercial sources, for example, J.T. Baker (Phillipsburg, PA), USB Corporation (Cleveland, OH).

Oils which may be suitable for use in compositions of the invention are the U.S. Food and Drug Administration (FDA) regulatory approved oils for injection in humans.

In some embodiments, the hydrophobic dispersant comprises a vegetable oil.

In some embodiments, the hydrophobic dispersant comprises one or more oils selected from mineral oil and vegetable oil, e.g., soybean oil, canola oil, and/or olive oil.

In some embodiments, the hydrophobic dispersant comprises soybean oil. The term "soybean oil" used herein may refer broadly to any raw soybean oil or processed soybean oil that contains at least one form of triglyceride or its derivative suitable for the polymerization reaction of the present invention.

In some embodiments, the hydrophobic dispersant comprises olive oil. The term "olive oil" refers to vegetable oils obtained from fruit of plants e.g., as classified in the genus Olea of the family Oleaceae (e.g., Olea europaea Linne (Oleaceae)). Olive oil may be obtainable from fruit by using a known pressing method and a known refinement method. For example, the olive oil may be obtainable by collecting oil with a cold-pressed method (oil collection without heating) performed immediately on mature fruits, performing mechanical filtering or centrifugation, and then performing a refinement process.

In some embodiments, the hydrophobic dispersant comprises almond oil. The term "almond oil" refers to vegetable oils obtained from kernels of plants e.g., classified in the genus Prunus of the family Rosaceae (e.g., a variety of Prunus amygdalus Batsch (Rosaceae), sweet almond). Almond oil may be obtainable from such kernels by using a known pressing method and a refinement method.

In some embodiments, the hydrophobic dispersant comprises wheat germ oil.

The term "wheat germ oil" refers to vegetable oils obtainable from germ of plants e.g., classified in the genus Triticum of the family Gramdneae (e.g., Triticum aestivum Linne (Gramdneae)). Wheat germ oil may be obtainable from such germ by using a known pressing method and a refinement method.

In some embodiments, the hydrophobic dispersant comprises camellia oil.

The term "camellia oil" refers to vegetable oils obtained from seeds of plants e.g., classified in the genus Camellia of the family Theaceae. Camellia oil may be obtainable from such seed by using a known pressing method and a known refinement method. For example, the camellia oil may be obtainable by grinding seeds dried in the sun or artificially, followed by steaming, compressing, and filtering the resultant for refinement.

In some embodiments, the hydrophobic dispersant comprises corn oil.

The term "corn oil" refers to vegetable oils obtainable from germ of plants classified e.g., in the genus Zea of the family Gramineae (e.g., Zea mays Linne (Gramineae)). Corn oil may be obtainable from such germ by using a known pressing method and a known refinement method. For example, the corn oil may be obtainable by selecting germs from grains, washing the germs with water followed by rapid heating and drying, compressing the resultant germs, and then extracting oil from the pomace with hexane.

In some embodiments, the hydrophobic dispersant comprises rape-seed oil.

The term "rape-seed oil" refers to vegetable oils obtained from seeds of plants classified in the genus Brassica of the family Cruciferae (e.g., Brassica campestris Linne subsp. napus Hooker filiuset Anderson var. nippo-oleifera Makino (Cruciferae)). Rape-seed oil may be obtainable from such seed by using a known pressing method and a known refinement method. For example, the rape-seed oil may be produced by heating and compressing seeds, performing dispersant extraction on the pomace, mixing the extracted oil with the compressed oil to produce raw oil, and refining the raw oil obtained.

In some embodiments, the hydrophobic dispersant comprises sunflower oil. The term "sunflower oil" collectively refers to vegetable oils obtained from seeds of plants classified in the genus Helianthus of the family Compositae (e.g., Helianthus annuus Linne (Compositae)). Sunflower oil may be obtainable from such seed by using a known pressing method and a known refinement method.

In some embodiments, the hydrophobic dispersant comprises cottonseed oil.

The term "cottonseed oil" refers to vegetable oils obtained from seeds of plants classified e.g., in the genus Gossypium of the family Malvaceae (e.g., Gossypium hirsutumLinne (Gossypium), or a plant classified in the same genus (Malvaceae)). Cottonseed oil may be obtainable from such seed by using a known pressing method and a known refinement method. For example, cottonseed oil may be obtainable by refining nonvolatile fatty oil obtained by performing a compression or extraction process on seeds.

In some embodiments, the hydrophobic dispersant comprises palm oil.

The term "palm oil" refers to vegetable oils obtained from seeds of plants classified in the genus Cocos of the family Palmae (e.g., Cocos nucifera Linne (Palmae)). Palm oil may obtainable from such seed by using a known pressing method and a known refinement method. For example, the palm oil may be obtained by further grinding the ground copra, steaming, compressing, and then removing suspended matter for refinement.

The inventors have found that compositions comprising some hydrophilic polyol, such as PEG 400 barely formed clot (as tested in the presence of saline) which may raise the concern of their hemostatic efficacy, as composition comprising PEG400 failed to form intact layer during the gelation test (see **Figure 1C).**

By "barely formed clot" it is meant that the clotting time is found to be above 20 min.

The term "clotting time" as used in the context of the present invention may be generally defined as the time required for the formation of a fibrin clot.

Accordingly, in some embodiments, the composition is substantially devoid of hydrophilic dispersant.

By "substantially devoid of" it is meant that the composition comprises less than 8 %, less than 5 %, less than 3 %, less than 1 %, or even completely absent of the relevant component (e.g., hydrophilic dispersant), for example, without limitation, water and polyols. For example, the composition may be substantially devoid of polyols. In some embodiments the composition is devoid of (i.e. being less than 1 %, or at times less than 0.5 %, by weight) PEG.

In some embodiments, the composition further comprises collagen.

The term "collagen" is intended to mean any known collagen of porcine, bovine or human origin, for example natural collagen, esterified collagen, such as methylated, ethylated or alternatively succinylated collagen, or one of its derivatives, which may or may not be heated, which may or may not be oxidized, or alternatively, for example, which may be crosslinked with another compound. For the purpose of the present disclosure, the term "natural collagen" is intended to mean collagen which has not been chemically modified, other than a possible treatment with e.g., pepsin in order to digest the telemeric peptides. Thus, for the purpose of the present invention, the term "non-denatured collagen" is intended to mean collagen which has not lost its helical structure.

A way of characterizing the texture of the composition is to measure the rheological properties, which involves the storage shear modulus G' and the shear loss modulus G" and the relation between the two. The term "storage shear modulus", G', may also be regarded as the elastic modulus, while the term "shear loss modulus", G", may be regarded as the viscous modulus.

Storage shear modulus and loss shear modulus may optionally be determined using a shear rheometer, for example, a strain-controlled rotational rheometer, at an indicated temperature and frequency (e.g., using procedures described in the Examples section herein).

A degree of viscoelasticity may optionally be characterized by a loss tangent (G"/G'), which is a ratio of a loss shear modulus (G") to storage shear modulus (G') and is a measure of the relative importance of the viscous to elastic contributions for a material at a given frequency, and may be evaluated at a given oscillation torque and a given temperature, such that: G' >G" - gel character and the elastic behavior dominates over the viscous behavior, i.e. the structure shows a certain rigidity; G">G'- liquid character: in this case, the viscous behavior dominates over the elastic behavior, and the sample shows the character of a liquid. The intersection (crossing point) of G' and G" is the yield point (or the crossing point; tau, $\tau$).

In some embodiments of any one of the embodiments described herein, the paste (according to any of the respective embodiments described herein) is characterized by yield point in a range of from about 0.1 to 15 Pa, at a temperature of 25 °C and frequency of 10 rad/sec. In some such embodiments, the yield point is in a range of from 0.1 to 13 Pa. In some such embodiments, the loss tangent is in a range of from 0.13 to 5 Pa.

As indicated in the Examples section that follows, the tau value for various samples was measured, showing that the force needed to push the samples forward is directly proportional to the collagen concentration and is reversely proportional to powder to oil ratio. Compared to the control (Surgiflo) the yield point of each sample is smaller, indicating that the range 1:1.5 to 1:1.7, and 0 to 20 % collagen can result in a formulation with the appropriate pushing force and gel character during the application.

As is further demonstrated in the Examples section that follows, the result of gelling time shows that the intervals between the water adding point and the crossing point (which represents the gelling time of the samples) for the sample of 1:1.7 blend to medium, with no collagen or 10 % collagen were both less than 3 min. The crossing point of samples of 1:1.7 blend to medium, with 20 % collagen cannot be seen within 10 min, indicating these formulations have a long gelling time, and may be less suitable for hemostatic applications.

Thus, in some embodiments of any one of the embodiments described herein, a paste (according to any of the respective embodiments described herein) is characterized by a gelling (also referred to as "gelation") time of less than 10 min upon contact with water. In some such embodiments, a paste (according to any of the respective embodiments described herein) is characterized by a gelling time of at most about 3 min upon contact with water. In some such embodiments, a paste (according to any of the respective embodiments described herein) is characterized by a gelling time of at most about 20 to 150 sec upon contact with water. In some such embodiments, a paste (according to any of the respective embodiments described herein) is characterized by a gelling time of at most about 30 to 120 sec upon contact with water.

In some embodiments, the composition, in any embodiment thereof, is characterized by a gelation time of less than 20 min, less than 19 min, less than 18 min, less than 17 min, less than 16 min, less than 15 min, less than 14 min, less than 13 min, less than 12 min, less than 11 min, less than 10 min, less than 9 min, less than 8 min, less than 7 min, less than 6 min, or less than 5 min.

In some embodiments, the composition in any embodiment thereof is characterized by a gelation time of about 15 min, about 14 min, about 13 min, about 12 min, about 11 min, about 10 min, about 9 min, about 8 min, about 7 min, about 6 min, or about 5 min, including any value and range therebetween.

In some embodiments, the composition in any embodiment thereof is characterized by a gelation time of 1 to 15, or 3 to 15, e.g., 1, 2, 3, 4, 5, 6, 7, 8, 9, 10, 11, 12, 13, 14, or about 15 min, including any value and range therebetween.

In some embodiments, the composition comprises water absorbing agent (e.g., ORC) and is characterized by a gelation time of less than 15 min, less than 14 min, less than 13 min, less than 12 min, less than 11 min, less than 10 min, less than 9 min, less than 8 min, less than 7 min, less than 6 min, or less than 5 min, while maintaining the superior adhesiveness of the composition (see Tables 3 and 8 below).

In some embodiments, the composition comprises water absorbing agent (e.g., ORC) and is characterized by a gelation time of about 10 min, about 9 min, about 8 min, about 7 min, about 6 min, or about 5 min, including any value and range therebetween, while maintaining the superior adhesiveness of the composition.

The term "gelation time" as used herein refers to the time it takes for the composition to lose flow as measured from the time when contacting an aqueous medium (e.g., blood or saline) *in vitro.* "Clotting time" as used herein refers to a similar process, *in vivo,* wherein other components, e.g. platelets, tissue factors may be involved.

Typically, at the gelation time the viscosity of the composition increases abruptly (e.g., at a shear rate of about 2 to 3 s$^{-1}$) to a value greater than 1000 cP.

In exemplary embodiments, the gelation time is determined when gel was formed and could be peeled as an intact layer and may be determined according to the yield point.

The term "gel", or any grammatical inflection thereof, relates to a viscous and/or solidlike material that can have properties ranging from soft and weak to hard and tough.

The gel can be a clot being a thick mass of coagulated liquid, especially blood.

In certain embodiments of the invention, composition may further include a hemostatic agent, or other biological or therapeutic compounds, moieties or species, including drugs and pharmaceutical agents.

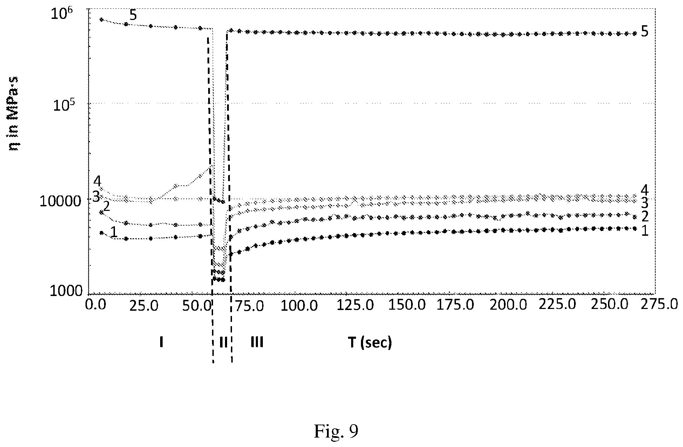

As is further demonstrated in the Examples section that follows upon evaluating the deformation and regeneration of different paste formulation (thixotropy test; see Figure 9) the sample without collagen showed very slow structural recovery, indicating that the sample will run away once applied on the wound site. The result also indicates that samples with 10 % collagen exhibited fast structural recovery, with the 20 % collagen sample exhibiting less desired recovery behavior.

Thus, in some embodiments, the composition further comprises collagen at a concentration of above 0 % up to about 20 %, by weight. In some embodiments, the composition further comprises collagen at a concentration of above 0 % up to about 10 %, by weight. In some embodiments, the composition further comprises collagen at a concentration of above 0 % up to less than about 20 %, e.g., about 10 %, about 11 %, about 12 %, about 13 %, about 14 %, about 15 %, about 16 %, about 17 %, about 18 %, or about 19 %, by weight, including any value and range therebetween.

In some embodiments, the composition is a hemostatic composition.

By "hemostatic composition" it is meant that composition may easily be applied to a site of need e.g., for achieving hemostasis in case of a puncture wound or a tissue gap.

The term "wound" includes damage to any tissue in a living organism.

Thus, the disclosed composition may be applied to a bleeding tissue, enabling to assist in hemostasis.

The term "tissue" refers to an association of cells and/or cell components united in carrying out a particular function. The cells in the tissue may be all of one type or of more than one type. The tissue can be an artificial tissue in which cells are grown to function in a similar manner as a tissue in a living organism. The tissue may be a human body tissue or an animal tissue.

In some embodiments, the tissue is a soft tissue. The term "soft tissues" as used herein relates to body tissue that is not hardened or calcified. This term especially relates to soft tissues that are vascularized and therefore may be a source of bleeding. Examples for such tissues include but are not limited to connective tissue (such as tendons, ligaments, fascia, skin, fibrous tissues, fat, and synovial membranes), muscles, internal organs, or blood vessel. In general, soft tissues are meant to exclude bone tissue.

In some embodiments, the tissue is a bone tissue.

"Hemostasis" (or "haemostasis") refers to the first stage of wound healing. It is a process which causes bleeding to stop. By "assist in hemostasis" is meant to help reduce or stop bleeding. By "applied to a bleeding tissue" it is meant to refer to e.g., a topical application of the composition at the bleeding site, e.g., at a surgical site to control bleeding. Control of bleeding is needed in various situations including treatment of wounds.

By "topical application" it is meant to refer to being applied locally to a part of the body.

The term "blood", or any grammatical inflection thereof, also includes blood fractions, such as plasma.

In some embodiments, the composition is for therapeutic use.

In some embodiments, the composition is applied to an injured tissue, e.g., a bleeding tissue. In some embodiments, the composition is present, e.g., as a one component, in a tube such as squeezable tube.

As used herein, the term "bleeding" refers to extravasation of blood from any component of the circulatory system. "Bleeding" thus encompasses unwanted, uncontrolled and often excessive bleeding in connection with surgery, trauma, or other forms of tissue damage, as well as unwanted bleedings in patients having bleeding disorders.

The term "trauma" is defined as an injury caused by a physical force; non-limiting examples include the consequences of vehicle accidents, gunshots and burns.

In another embodiment, the disclosed composition may be used in conjunction with a backing, pad, bandage, gauze, sponge, scaffold, or matrix to provide mechanical strength to cover the wound surface. In this case, the instant matrix is supported on a pad for ease of application or tamponade.

In some embodiments, the composition is sterile. Especially when handling blood products, the sterility issue is crucial, and specifically the issue of viral inactivation. In general, viral inactivation may be carried out by any number of methods, including solvent detergent, heat inactivation, irradiation, and nanofiltration. Typically, the standard for viral inactivation requires using two different methods. Additionally, FDA standard for sterility requires filtration. The term "preparation" refers to a physiologically acceptable suitable for therapeutic use.

The term "sterile" as used herein means having a low bioburden, effectively being germ-free, e.g., essentially or even absolutely being free from microorganisms, e.g., bacteria and viruses. Sterilization is the process of reducing the bioburden to an effectively germ-free level. A sterile liquid or paste is generally defined as a liquid or paste that has underwent sterile filtration.

As used herein, "thrombin" denotes an activated enzyme which results from the proteolytic cleavage of prothrombin (factor II). Thrombin may be produced by a variety of methods of production known in the art, and includes, but is not limited to, recombinant thrombin and plasma derived thrombin. Human thrombin is a 295 amino acid protein composed of two polypeptide chains joined by a disulfide bond. Both human and non-human (e.g., bovine) thrombin may be used within the scope of the present disclosure.

The origin for thrombin used in this invention may be from one or several sources including but not limited to: plasma (e.g., porcine plasma), recombinant bacteria and/or cells (Vu et al., 2016, J. Viet. Env. 8(1):21-25), whole blood (pooled from several donations or not) and/or blood fraction (that may be pooled from several donations, e.g., plasma). Thrombin is available by manufacturers such as Johnson and Johnson, Baxter and CSL Behring either as a standalone product, e.g., EVITHROM®, or as a component of a product e.g., EVICEL®, TISEEL®, Beriplast®, and the like.

In some embodiments, the thrombin is present at a concentration of 5 % to 10 %, or 7 % to 8 %, by weight of the blend. In some embodiments, the thrombin is present at a concentration of 5 %, 5.5 %, 6 %, 6.5 %, 7 %, 7.5 %, 8 %, 8.5 %, 9 %, 9.5 %, or 10 %, by weight of the blend, including any value and range therebetween.

The term "fibrinogen" without more is intended to include any type of fibrinogen. "Fibrinogen" refers to monomeric and dimeric fibrinogen molecules having the monomer structure (AαBβγ), hybrid molecules, and variants thereof, whether naturally occurring, modified, or synthetic. The term "fibrinogen" may refer to fibrinogen from humans, but may include fibrinogen of any species, especially mammalian species, such as fibrinogen produced from porcine plasma.

In some embodiments, the fibrinogen is present at a concentration of 60 % to 95 %, or 70 % to 90 %, by weight of the blend. In some embodiments, the thrombin is present at a concentration of 60 %, 61 %, 62 %, 63 %, 64 %, 65 %, 66 %, 67 %, 68 %, 69 %, 70 %, 71 %, 72 %, 73 %, 74 %, 75 %, 76 %, 77 %, 78 %, 79 %, 80 %, 81 %, 82 %, 83 %, 84 %, 85 %, 86 %, 87 %, 88 %, 89 %, 90 %, 91 %, 92 %, 93 %, 94 %, or 95 %, by weight of the blend, including any value and range therebetween.

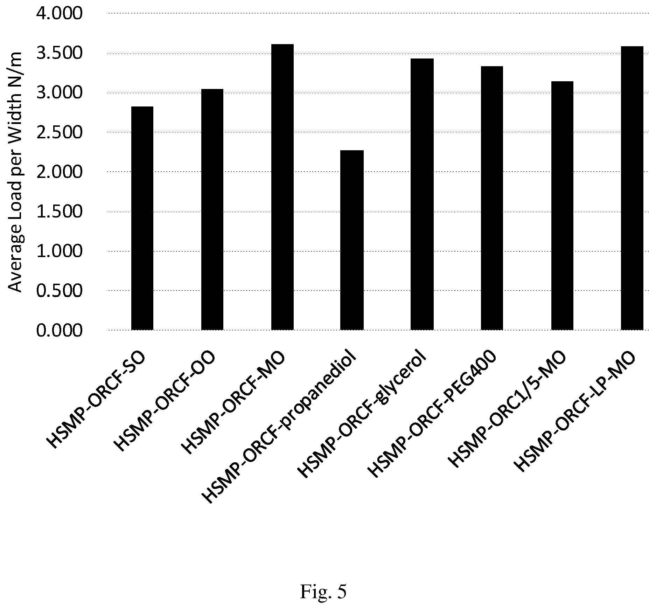

Reference is made again to **Figure 1A** showing that for that some dispersants, for instance, glycerol, although exhibiting high adhesive property (see **Figure 5**), failed to render the composition the desired flowability.

Accordingly, in some embodiments, the composition is substantially devoid of hydrophilic dispersant selected from polyethylene glycol (PEG; e.g., small molecular weight of PEG such as PEG 400) and/or glycerol.

In some embodiments, the composition comprises water absorbing agent, also referred to herein as: "hydraulic agent", or "siphonic agent".

In some embodiments, the water absorbing agent comprises oxidized cellulose (OC). For example, the oxidized cellulose may comprise oxidized regenerated cellulose (ORC).

The term "water absorbing agent" refers to a material that absorbs water from a medium, e.g., forming a hydrate. As used herein, the term "hydrate" refers to a combination of water with a substance. In some embodiments, the water absorbing agent (e.g., ORC) quickly absorbs water upon contact with an aqueous medium, so that the water % in the composition raises by 2 % to 50 % within 5 min. In some embodiments, the water absorbing agent (e.g., ORC) quickly absorbs water upon contact with an aqueous medium, thereby reducing the gelation time compared to a composition not comprising the water absorbing agent.

The term "oxidized cellulose" (or "OC") refers to a cellulose derivative in which at least some of the primary alcohol groups, e.g., on the carbon 6 of the anhydroglucose unit is oxidized to a carboxylic acid, and is optionally functionalized. OC may include materials, products, articles, or compositions comprising or consisting essentially of OC, e.g., a dressing, fibrin glue, synthetic glue, pad, matrix, powder, tab, pill, suture, fiber, stent, implant, scaffold, solution, gel, wax, gelatin, and the like.

In some embodiments, the OC (e.g., ORC) is in the form of a plurality of fibers. In some embodiments, the OC (e.g., ORC) is in the form of a plurality of fibers having a median size below 80 $\mu$m. In some embodiments, the OC (e.g., ORC) is in the form of a plurality of fibers having a median size ranging from 50 to 100 $\mu$m, or 60 to 80 $\mu$m, e.g., 50, 60, 70, 80, 90, 100 $\mu$m, including any value and range therebetween.

OC may be produced by applying an oxidizing agent on cellulose. The oxidizing agent may be selected from, without being limited thereto, chlorine, hydrogen peroxide, peracetic acid, chlorine dioxide, nitrogen dioxide, persulfates, permanganate, dichromate-sulfuric acid, hypochlorous acid, hypohalites, periodates, or any combination thereof, and/or a variety of metal catalysts. Oxidized cellulose may contain carboxylic acid, aldehyde, and/or ketone groups, instead of, or in addition to, the original hydroxyl groups of the starting material, cellulose, depending on the nature of the oxidant and reaction conditions.

The term "contact" is used in its broadest sense and refers, for example, to any type of combining action which brings e.g., the hemostatic composition into sufficiently close proximity with water, aqueous solution, or blood, such that a clot or gel can be formed.

In some embodiments, the composition is substantially devoid of water absorbing agent such as ORC, as it also appears that the addition of ORC might decrease the adhesiveness property (T-peel force) (see e.g., Table 8 in the Examples section that follows).

In some embodiments, the water absorbing agent (e.g., ORC), if not absent, is present at a concentration of up to 3 %, up to 8 %, up to 10 %, up to 15 %, up to 20 % or up to 25 %, e.g., in the range of 3 to 15%, by weight of the blend. In some embodiments, the water absorbing agent is present at a concentration of 5 to 25 % by weight of the blend.

In some embodiments, the water absorbing agent is present at a concentration of 5 to 18 % by weight of the blend.

In some embodiments, the water absorbing agent is present at a concentration of 3 %, 4 %, 5 %, 6 %, 7 %, 8 %, 9 %, 10 %, 11 %, 12 %, 13 %, 14 %, 15 %, 16 %, 17 %, 18 %, 19 %, 20 %, 21 %, 22 %, 23 %, 24 %, or 25 %, by weight, including any value and range therebetween. In some embodiments, the water absorbing agent is present at a concentration of 8 % to 18 %, by weight.

In some examples, the fibrinogen may be present at a concentration of e.g., 60 % to 85 %, by weight of the blend, the thrombin may be present at a concentration of e.g., 5 to 10 %, by weight of the blend, and, in another example, the wetting agent may be present at a concentration of e.g., 5 to 18 %, by weight of the blend.

In further embodiments of the present invention, the disclosed composition may be combined with various additives to further improve the hemostatic properties, wound healing properties, and handling properties.

Utilizing additives known to these skilled in the art, include, for example: hemostatic additives such as gelatin, collagen, cellulose, chitosan, polysaccharides, starch; biologics based hemostatic agents as exemplified by thrombin, fibrinogen, and fibrin. Additional biologics hemostatic agents include, without limitation, procoagulant enzymes, proteins and peptides.

In some embodiments, the blend may be, for example, high-shear mixed, spray-dried mixing, or a combination thereof.

The term "spray drying" is used herein in a broad sense, which include, without being limited thereto, processes for transforming a solid dissolved or suspended in a liquid into a powdery. Typically, spray drying is a drying method used to create powder from a solution, suspension or emulsion that is atomized through a spray nozzle in a hot airflow and dried instantly. Spray drying process for producing thrombin and fibrinogen powders, which maintain their activity may be controlled by several process parameters, among them are: column air flow and temperatures, nozzle size and atomizing air flow rate, and material flow rate. Non-limiting exemplary parameters used in order to establish a robust process to produce a blend of thrombin and fibrinogen (e.g., in a powder form) which maintains its activity, low water content, high solid yield and desired powder particle size and distributions, are provided in the Examples section below.

The term "high-shear mixing" generally means a turbulent-flow type of mixing. In some embodiments, the high-shear mixing is characterized by speed of mixing blade and/or speed of cutting blade ranging from: 150 to 500 rpm.

Additionally, or alternatively, other methods may be used for obtaining dry composition, such as, without limitation, lyophilization of the fibrinogen solution and/or thrombin solution, followed by micronization. Typically, following lyophilization of the solution, a porous and spongy solid material, referred to as a "cake" (may also be referred to herein as "solid composition") is obtained.

The term "micronization" is used when the particles that are produced e.g., from the solid composition, are only a few micrometers in diameter. Micronization can be achieved by processes including, but not limited to, jet milling, pearl-ball milling, high-pressure homogenization, the RESS process (Rapid Expansion of Supercritical Solutions), the SAS (Supercritical Anti-Solvent) method, or the PGSS (Particles from Gas Saturated Solutions) method. Typically, but not exclusively, micronization results in a 30 to 400- fold size reduction of the protein powder from its original size.

In some embodiments, the blend is characterized by a density of 0.2 to 0.4 gr/ml, or 0.25 to 0.35 gr/ml. Thus, in some embodiments, the blend is characterized by a density of 0.2, 0.25, 0.3, 0.35, or 0.4 gr/ml, including any value and range therebetween.

For example, in some embodiments, the blend is high-shear mixed and is characterized by a density of 0.3 to 0.4 gr/ml. Thus, in some embodiments, the blend is high-shear mixed and is characterized by a density of 0.3, 0.35, or 0.4 gr/ml, including any value and range therebetween. In further embodiments, the blend is spray-dried mixed (without following high shear mixing) and is characterized by a density of 0.2 to 0.3 gr/ml, e.g., 0.2, 0.25, or 0.3 gr/ml, including any value and range therebetween.

In some embodiments, the particles of the blend are characterized by a median particles size of below 270 $\mu$m, below 260 $\mu$m, or below 250 $\mu$m. In some embodiments, the particles of the blend are characterized by a median particle size of below above 80 $\mu$m, above 90 $\mu$m, or below 100 $\mu$m.

Reference is made to **Figure 2** showing that particles sized in the range of 106 to 250 provide a quicker gelation time as compared to larger particles.

In some embodiments, the particles of the blend are characterized by a median particle size of about 100 to about 270 $\mu$m. In some embodiments, the particles of the blend are characterized by a median particle size of about 106 to about 250 $\mu$m. In some embodiments, the particles of the blend are characterized by a median particle size of about 100 $\mu$m, about 110 $\mu$m, about 120 $\mu$m, about 130 $\mu$m, about 140 $\mu$m, about 150 $\mu$m, about 160 $\mu$m, about 170 $\mu$m, about 180 $\mu$m, about 190 $\mu$m, about 200 $\mu$m, about 210 $\mu$m, about 220 $\mu$m, about 230 $\mu$m, about 240 $\mu$m, about 250 $\mu$m, about 260 $\mu$m, or about 270 $\mu$m, including any value and range therebetween.

In some embodiments, the composition is homogeneous. As used herein, by "homogeneous" it is meant to refer to a uniform composition and texture throughout, that is, the blend is dispersed substantially evenly throughout the oily medium. Herein, "dispersed substantially evenly" is meant that the concentration of the blend within the hydrophobic medium varies within less than ± 30 %, less than ± 20 %, less than ± 10 %, less than ± 5 %, by weight, throughout the oil. Typically, term "homogeneous" refers to a uniform appearing product (e.g., paste) which withstands a liquid/solid phase separation (e.g., less than 4 % by weight) at 8 to 40 °C, 10 to 30 °C or 20 to 30 °C.

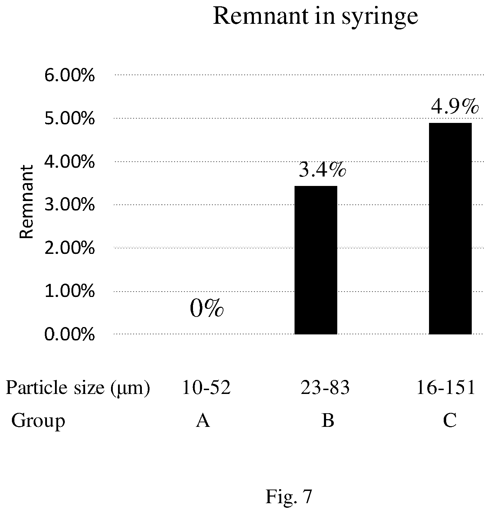

Phase separation may be detected by methods known in the art, e.g., as described in the Examples section below (see **Figure 7**).

In some embodiments, the composition is a storage stable composition. The term "storage stable" is to be understood as referring to the formulation, composition, typically being present in a container e.g., vial, or syringe that is stable under preselected storage conditions (see below), including pre-selected storage temperature, pre-selected physical state of the formulation. Stability can be determined by methods known in the art, e.g., by testing the absence of visible aggregations, remnant and/or fibrin clots in the formulation under the preselected storage condition, for example, when the composition is in liquid or pasty form. Further, in accordance with the present disclosure, when referring to a stable sealant composition or formulation it is to be understood as one that, upon use, has an effective clotting time irrespective of the formulation's storage conditions, e.g., the sealant formulation clots at essentially the same time period irrespective of whether it was stored at room temperature (e.g., 8 to 40 °C) or at even lower temperatures (e.g., below 8 °C).

The terms "stable", and "stability" when referring to the disclosed paste, mean substantially an absence of fibrin polymerization/clotting in the paste before it passes through and/or contacts aqueous medium, e.g., saline or blood, and at the same time, after certain duration at a certain temperature remain at least 70 % active, that is, capable of forming a fibrin clot.

In some embodiments, the pre-selected storage conditions comprise storage at room temperature (e.g., 10 to 30 °C) and the sealant formulation is stable for at least 5 minutes, at times for at least 15 minutes, or for at least 1 hour, for at least a day, at least 2, 3, 4, 5, 6, 7, 8, 9, 10, 11, 12, 13, 14, 15, 16, 17, 18, 19, 20, 21, 22, 23, 24, 25, 26, 27, 28, 29, or 30 days, at times for e.g., 1, 2, 3, 4, 5, 6, 7, 8, 9, 10, 11, 12, 13, 14, 15, 16, 17, 18, 19, 20, 21, 22, 23, or even 24 months, or more, including any value and range therebetween.

In some embodiments, less than 25 %, less than 24 %, less than 23 %, less than 23 %, less than 22 %, less than 21 %, or less than 20 %, by weight, remnant is detected after 30 days.

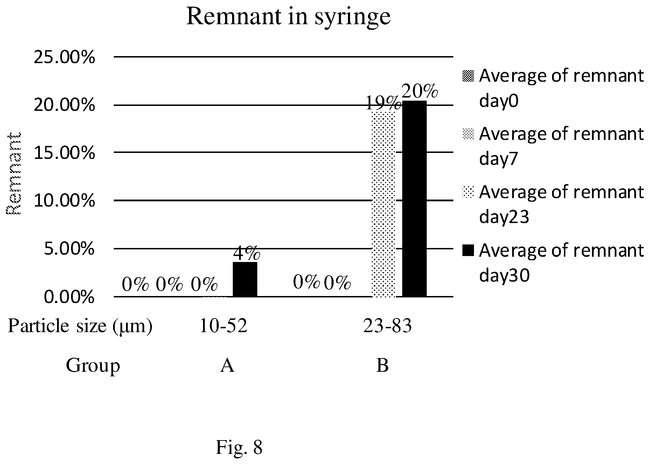

Reference is made to **Figures 7** and **8** showing that in exemplary embodiments, pasty composition comprises blend having smaller particles size (e.g., at least 90 % ranging from 10 to 52 micron) and provides superior homogeneity and stability. During a period of 30 days of storage, the blend with small particles (10-52 $\mu$m) exhibited less than 5% difference in remnant concentration (remnant was detected by doing an extrusion test using the Instron testing system), while blend with larger particles (23-83 $\mu$m) showed up to 20 % difference in remnant concentration. Taken together, the larger the particle size and the longer it was stored, the more remnant might be left within the syringe.

In some embodiments, the pasty composition comprises blend having particles size ranging from 10 to 52 micron, and less than 10 %, less than 9 %, less than 8 %, less than 7 %, less than 6 %, less than 5 %, or less than 1 %, by weight, remnant is detected after 30 days.

In some embodiments, the particles of the blend are characterized as being homogenous and stable with median particles' size of below 110 $\mu$m, e.g., about 10 to about 100 $\mu$m. In some embodiments, the particles of the blend are characterized by a median particles' size of about 10 to about 60 $\mu$m. In some embodiments, the particles of the blend are characterized by a median particles' size of about 10 $\mu$m, about 11 $\mu$m, about 12 $\mu$m, about 13 $\mu$m, about 14 $\mu$m, about 15 $\mu$m, about 16 $\mu$m, about 17 $\mu$m, about 18 $\mu$m, about 19 $\mu$m, about 20 $\mu$m, about 21 $\mu$m, about 22 $\mu$m, about 23 $\mu$m, about 24 $\mu$m, about 25 $\mu$m, about 26 $\mu$m, about 27 $\mu$m, about 28 $\mu$m, about 29 $\mu$m, about 30 $\mu$m, about 31 $\mu$m, about 32 $\mu$m, about 33 $\mu$m, about 34 $\mu$m, about 35 $\mu$m, about 36 $\mu$m, about 37 $\mu$m, about 38 $\mu$m, about 39 $\mu$m, about 40 $\mu$m, about 41 $\mu$m, about 42 $\mu$m, about 43 $\mu$m, about 44 $\mu$m, about 45 $\mu$m, about 46 $\mu$m, about 47 $\mu$m, about 48 $\mu$m, about 49 $\mu$m, or about 50 $\mu$m, including any value and range therebetween.

In some embodiments, the particles of the blend are characterized by D10 ranging from 20 to 60, or 30 to 50 $\mu$m. In some embodiments, the blend is characterized by D10 of 20, 30, 40, or 50 $\mu$m, including any value and range therebetween.

In some embodiments, the particles of the blend are characterized by a median particle size of about 106 to about 250 $\mu$m and the blend is characterized by D10 ranging from 20 to 60, or 30 to 50 $\mu$m, e.g., 20, 30, 40, or 50 $\mu$m, including any value and range therebetween.

In some embodiments, the particles of the blend are characterized by high homogeneity and stability, and the particles of the blend are characterized by D10 ranging from 5 to 20, or 8 to 15 $\mu$m. In some embodiments, the blend is characterized by D10 of 8, 10, 12, or 15 $\mu$m, including any value and range therebetween.

In some embodiments, the particles of the blend are characterized by D50 ranging from 130 to 150, e.g., 130, 135 140, 145, or 150 $\mu$m, including any value and range therebetween. In some embodiments, the particles of the blend are characterized by a median particle size of about 106 to about 250 $\mu$m and/or the blend is characterized by D50 ranging from 130 to 150, e.g., 130, 135, 140, 145, or 150 $\mu$m, including any value and range therebetween.

In some embodiments, the particles of the blend are characterized by increased homogeneity and stability, and the particles of the blend are characterized by D50 ranging from 20 to 40 e.g., 20, 25, 30, 35, or 40 $\mu$m, including any value and range therebetween.

In some embodiments, the blend is characterized by particles' size having D90 ranging from 280 to 340, e.g., 280, 290, 300, 310, 320, 330, or 340 $\mu$m, including any value and range therebetween.

In some embodiments, the particles of the blend are characterized by increased homogeneity and stability, and the particles of the blend are characterized by a median particles' size of about 8 to about 60 $\mu$m and are further characterized by D90 ranging from 40 to 60, e.g., 40, 45, 50, 55, or 60 $\mu$m, including any value and range therebetween.

The particles' size of D50 refers to a median diameter corresponding to a volume-based accrued value of 50 volume %. The particle diameter D10 refers to a particle diameter corresponding to a volume-based accrued value of 10 volume %. The particles' size of D90 refers to a median diameter corresponding to a volume-based accrued value of 90 volume %.

The particle's diameter refers to a particle diameter in which the particle is approximated as having a spherical shape and may be measured using methods known in the art, for example, a laser diffraction particle analyzer (e.g., that manufactured by MALVERN, MASTER SIZER).

In an aspect of the present disclosure, the disclosed composition in any aspect or embodiment thereof is for use in a method for preparing a fibrin adhesive sealant in/on an injured tissue of a subject, e.g., by applying the disclosed composition in any aspect and/or embodiment thereof on a surface of the tissue.

In an aspect of the present disclosure, there is provided a composition for preparing a fibrin adhesive sealant in/on an injured tissue of a subject, e.g., by applying the disclosed composition in any aspect and/or embodiment thereof on a surface of the tissue.

In the context of the present invention, the term "sealant", also referred to as "fibrin sealant", and "biological glue", is to be understood as a single/one component adhesive/glue/hemostat, e.g., originates or being derived from the disclosed composition, having ingredients that upon contact with, or in proximity to, a tissue and/or blood, reacts to subsequently form a clot, acting as a tissue adhesive, and thereby prevents, reduces, or stops bleeding, joins structures and/or seals physiological leaks, e.g., of cerebrospinal fluids (CSF), lymph, bile, gastrointestinal (GI) content, air leak from lungs etc. In some embodiments, the sealant formulation also comprises one or more therapeutics such that upon natural degradation of the clot formed in the body, the therapeutic is released. The therapeutic may be, without being limited thereto, drug(s), such as antibiotics, analgesics, anti-inflammatory drugs, cancer drugs etc., cells including, for example, any type of stem cells e.g., Embryonic Stem (ES) cells, adult stem cells, Pluripotent Stem Cells (iPSCs) etc. from human or other origin.

It is also possible that the disclosed composition comprises components which encourage the formation of the clot, such as Ca, Factor VIII, fibronectin, vitronectin, von Willebrand factor (vWF) which can be provided as a separate component or formulated with the blend or the paste.

As used herein, the term "subject" shall mean any animal including, without limitation, a human, a mouse, a rat, a rabbit, a non-human primate, or any other mammal. In some embodiments, the subject is human, e.g., a human patient. The subject may be male or female.

As used herein, the term "injured tissue", (or "damaged tissue") refers to disruption of the normal continuity of structures caused by a physical (e.g., mechanical) force such as in incisions caused by surgery, a biological (e.g., thermic or actinic force), or a chemical means. The term "injured tissue" also encompasses contused tissues, as well as incised, stab, lacerated, open, penetrating, puncture, injuries caused e.g., by ripping, scratching, pressure, and biting. The term "injured tissue" also encompasses wounds and bleeding sites as described hereinthroughout.

Thus, in some embodiments, the disclosed composition in any embodiment thereof may be used applied in difficult-to-reach bleeding sites, for example during minimally invasive surgeries (MIS) such as, e.g., endoscopy. One of the common forms of endoscopy is laparoscopy which is minimally-invasive inspection and surgery inside the abdominal cavities.

The term "surgery" also encompasses the time during or after surgical or diagnostical procedures. Further non-limiting examples of procedures include neurosurgery, abdominal surgery, cardiovascular surgery, thoracic surgery, head and neck surgery, pelvic surgery and skin and subcutaneous tissue procedures. For at least one of these situations, the composition of the invention may serve as a suitable sealant, which permits adhesion to the bleeding wound, and thus addresses hemostasis without being depended by the condition of the wounded tissue, e.g., severity of bleeding. The formation of the sealant occurs in a relatively short period of clotting time subsequent applying the disclosed composition, in any aspect and/or embodiment thereof, on an injured tissue. Typically, using after 1 to 3 min tamponade, the incomplete clot may form which enables to stop bleeding, but more time may be needed for clotting reacting up to the paste surface thus forming a complete clot.

Herein, the adhesion strength may be measured by the pull-force strength required to break the contact between the wound dressing and the tissue.

In some embodiments, the disclosed composition upon application thereof onto the wound provides an adhesive sealant, characterized by superior adhesiveness to the tissue.

Herein "superior adhesiveness" means at least 3.5 N/m, at least 4 N/m, or at least 5 N/m as tested using a T-peel test.

In some embodiments, "superior adhesiveness" means 3.5 N/m to about 5.5 N/m, e.g., about 3.5 N/m, about 4 N/m, about 4.5 N/m, about 5 N/m, about 5.5 N/m, including any value and range therebetween, as tested using a T-peel test.

The unit "N/m" (i.e. average load/width) as used herein refers to peel strength when the tested body and the sealing sheet are bonded together, with a bonding portion between the tested body and the sealing sheet being cut to a width of a certain mm and a T peel test being made on this cut piece as a test sample. This test is referred to as a "T-peel" test because as the two adherends are pulled apart, they form the shape of a "T".

The T-peel test as used herein is a test based on a measurement according to the ASTM F2256-05(2015) entitled: "Standard Test Method for Strength Properties of Tissue Adhesives in T-Peel by Tension Loading", with the modification of using silicon sheet instead of porcine skin.

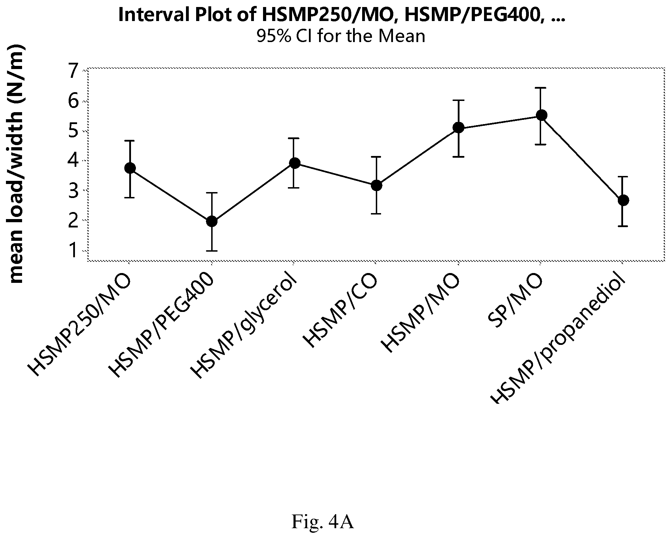

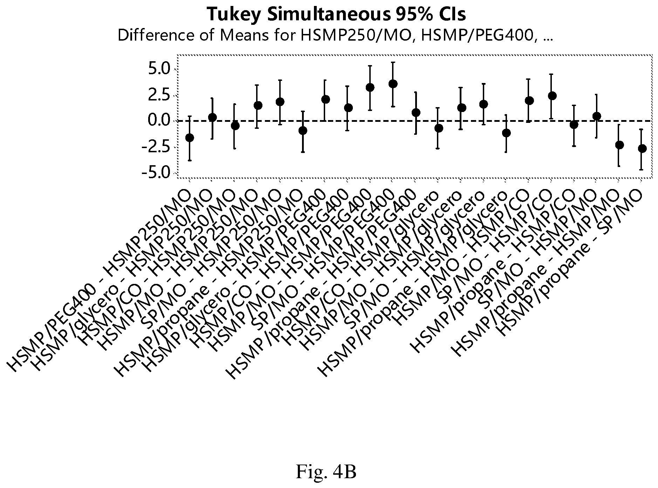

Reference is made to **Figures 4** and **5** showing that the compositions comprising oil, such as mineral oil, provide a sealing exhibiting a superior adhesiveness.

In another aspect of the present invention, there is provided a composition for use in a method of treating a wound comprising the step of applying (e.g., contacting) the disclosed composition in any aspect and/or embodiment thereof onto and/or into the wound of a subject in a need thereof.

By "treating a wound" it is further meant to encompass reducing blood loss at a bleeding site of a tissue, e.g., in a patient undergoing surgery.

Accordingly, in some embodiments, the method is for reducing blood loss at a bleeding site of a tissue and/or making a sealant layer in/on such a tissue, e.g., in a patient undergoing surgery, the method comprising contacting the disclosed composition in an embodiment thereof with the bleeding site. Optionally, the method comprises first applying on such a tissue an aqueous solution, such as saline, and thereafter applying on the aqueous solution the disclosed composition, so as to promote a fast clotting time.

In another aspect, the present invention further provides a hemostatic kit comprising a container containing the herein disclosed composition in an embodiment thereof, e.g., composition comprising a blend of fibrinogen, thrombin, wherein the ratio of the blend to the hydrophobic dispersant ranges e.g., from 0.3:1, 0.4:1, or 0.5:1 to below 0.9:1 (w/w). In some embodiments of the kit, the composition is in the form of a paste at around room temperature.

Any aspect and embodiment of the hereinthroughout disclosed composition may be incorporated to the aspect and embodiments of the kit, including embodiments of the composition, the hydrophobic dispersant, and/or the blend.

Alternatively, present invention provides a hemostatic kit comprising a container containing the herein disclosed blend of fibrinogen and thrombin, and another container comprising hydrophobic dispersant. In such embodiments, the kit may further contain a measuring means, e.g., a measuring cylinder, to measure the volume of the hydrophobic dispersant, such that ratio of the blend to the hydrophobic dispersant can be determined to be e.g., from 0.3, 0.4, or 0.5 to below 0.9. Additionally or alternatively, the hemostatic kit may comprise a syringe containing the blend and a syringe containing the hydrophobic dispersant.

In some embodiments, at least one of the containers in the kit is a pre-filled syringe. In some embodiments, a syringe is provided in addition to the containers of the kit. In some embodiments, the container is in a specific type, such as a vial or an applicator.

In some embodiments, at least one of the containers in the kit is a pre-filled syringe. In some embodiments, a syringe is provided in addition to the container(s) of the kit. The term "container" may refer to any generic structure such as a vessel or a vial, that may contain e.g., paste.

The kit may be applied using an applicator device which may be used for administering several and sequential injections of the composition. In one embodiment, the applicator device enables multiple injections of a fixed-dose of the mixed components on a 2-D surface of a tissue while moving the device. In one embodiment, the applicator has a syringe with an injection needle, which is optionally automatically retracted from the patient's skin after the injection is completed without the need for the administrator to lift the device upward from the injection surface. In one embodiment, the kit may be used for the administration of a sealant.

The hemostatic kit of the invention may be a kit for use in reducing, preventing or stopping blood flow, e.g., in open wounds, and it may be used for reducing, preventing or stopping blood flow during a procedure, such as during, before, or after a surgical procedure such as, for example, laparoscopic surgery, neurosurgery, abdominal surgery, cardiovascular surgery, thoracic surgery, head and neck surgery, pelvic surgery and skin and subcutaneous tissue procedures. The kit may be used for reducing or preventing blood flow from the skin, or in internal organs.

In one embodiment, this kit may be stored at room temperature, such as in a temperature in the range of 8 to 40 °C, or at lower temperatures.

In some embodiments of any aspect of the kit or the composition disclosed herein, the blend may comprise an additive e.g., calcium salt and/or one or more excipients, e.g., selected from, without being limited thereto, one or more amino acids, albumin, saccharides, and/or saccharide derivatives.

The term "additive" is meant to be understood as any substance that can be added to a composition, and may also include an active additive such as calcium salt as described below.

The term "excipient" as used herein denotes a non-active or non-therapeutic agent added to a pharmaceutical composition e.g., to provide a desired consistency or stabilizing effect.

Calcium is an important element in the clotting cascade, and may be needed for activation of factor XIII into factor XIIIa, which cross-links and stabilizes fibrin to generate an insoluble clot.

Accordingly, in some embodiments of any aspect of the disclosed kit and/or composition, the blend further comprises additive such as, without limitation, calcium. Calcium used with the invention may be in the form of a salt, e.g., calcium chloride salt. Alternatively, additional salts may be used, such as calcium acetate and/or calcium citrate. In the kit, the calcium salt may be provided in the composition comprising the blend. Alternatively, the excipient(s), and/or the calcium salt, may be provided in the kit in a separate container, or the excipient(s), and/or the calcium salt, may be provided in the kit in the same container comprising the blend component.

In some embodiments of the kit or the composition, the calcium salt, e.g., calcium chloride ($CaCl_2$) is present at a concentration of 0.5 % to 4 %, or 2.5% to 3.5 %, by weight of the blend. In some embodiments, the calcium salt, e.g., calcium chloride is present at a concentration of 0.5 %, 1 %, 1.5 %, 2 %, 2.5 %, 3 %, 3.5 %, or 4 %, by weight of the blend, including any value and range therebetween.

In some embodiments of any aspect of the kit and/or compositions, the blend further comprises an excipient selected from amino acids e.g., lysine. In some embodiments of any aspect of the kit and/or compositions, the blend comprises OC and lysine.

In some embodiments, the lysine is present at a concentration of 4 % to 8 %, or 5 % to 7 %, by weight of the blend. In some embodiments, the lysine is present at a concentration of 4 %, 4.5 %, 5 %, 5.5 %, 6 %, 6.5 %, 7 %, 7.5 %, or 8 %, by weight, including any value and range therebetween.

In another aspect, there is provided a method of making the disclosed composition in any embodiment thereof.

The method may comprise providing a blend of fibrinogen, thrombin, wherein the blend is in the form of a plurality of particles, and adding to the blend a hydrophobic dispersant, in a ratio of blend to the hydrophobic dispersant ranging from 0.5:1 to below 0.9:1 (w/w), so as to provide a paste at at-least one temperature in the range of 10 °C to 37 °C.

In some embodiments, the blend is aggregated e.g., by compacting a blend of fibrinogen, and thrombin e.g., made by high shear mixing, and also one or more active components and/or excipients as described herein (e.g., calcium salt and/or lysine) are present in the blend or in the composition.

In one embodiment of the invention, the method further comprises the step of subjecting the blend to drying, milling; and sieving, thereby obtaining hemostatic aggregates.

Aggregates may be made by a step of: humidifying the hemostatic powder composition; compacting, e.g. by roller and/or slugging, the powder to form hemostatic aggregates; spray drying; high-shear mixing; dehumidifying; milling. The step may further comprise sieving the hemostatic aggregates; and optionally dosing the resulting hemostatic aggregates into storage containers or into delivery devices.

Embodiments of spray drying and high-shear mixing are described hereinthroughout.

The powder can be roller compacted or slugging compacted and then may be subjected to pre-breaking, dehumidification, and subsequently followed by a step of final milling. The resulting aggregates may be selected to a targeted hemostatic aggregate fraction, e.g., by sieving for desired size and/or distribution separation (e.g., below 250 $\mu$m, or below 110 $\mu$m, in a defined range of D10, D50 and D90).

The hemostatic blend, intended for dosing, may have moisture content e.g., when measured by "loss on drying" method of less than about 8 %, less than about 5 %, or less than 2 %, by weight.

The term "about" as used herein means that values that are 10% above or below the indicated value are also intended to be included. Generally, all values in this application are intended to include the term "about".

The terms "comprises", "comprising", "has", "having", "contain", "containing", and their conjugates mean "including but not limited to". The term "consisting of" means "including and limited to". The term "consisting essentially of" means that the composition, method or structure may include additional ingredients, steps and/or parts, but only if the additional ingredients, steps and/or parts do not materially alter the basic and novel characteristics of the claimed composition, method or structure.

The word "exemplary" is used herein to mean "serving as an example, instance or illustration". Any embodiment described as "exemplary" is not necessarily to be construed as preferred or advantageous over other embodiments and/or to exclude the incorporation of features from other embodiments.

The word "optionally" is used herein to mean "is provided in some embodiments and not provided in other embodiments". Any particular embodiment of the invention may include a plurality of "optional" features unless such features conflict.

As used herein, the singular form "a", "an" and "the" include plural references unless the context clearly dictates otherwise. For example, the term "a compound" or "at least one compound" may include a plurality of compounds, including mixtures thereof.

Throughout this application, various embodiments of this invention may be presented in a range format. It should be understood that the description in range format is merely for convenience and brevity and should not be construed as an inflexible limitation on the scope of the invention. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 3, 4, 5, and 6. This applies regardless of the breadth of the range.

Whenever a numerical range is indicated herein, it is meant to include any cited numeral (fractional or integral) within the indicated range. The phrases "ranging/ranges between" a first indicate number and a second indicate number and "ranging/ranges from" a first indicate number "to" a second indicate number are used herein interchangeably and are meant to include the first and second indicated numbers and all the fractional and integral numerals therebetween.

As used herein the term "method" refers to manners, means, techniques and procedures for accomplishing a given task including, but not limited to, those manners, means, techniques and procedures either known to, or readily developed from known manners, means, techniques and procedures by practitioners of the chemical, pharmacological, biological, biochemical and medical arts.

As used herein, the term "treating" in the context of medical treatment includes abrogating, substantially inhibiting, slowing or reversing the progression of a condition, substantially ameliorating clinical or aesthetical symptoms of a condition or substantially preventing the appearance of clinical or aesthetical symptoms of a condition.

As used herein, the terms "controlling", "preventing", or "reducing", which may be used herein interchangeably in the context of the bleeding, including any grammatical inflection thereof, indicate that the rate of the blood extravagated is essentially nullified or is reduced e.g., by 10 %, at least 20 %, at least 30 %, at least 40 %, at least 50 %, at least 60 %, at least 70 %, at least 80 %, at least 90 %, or even by 100 %, of the initial rate of bleeding, compared to situation lacking the contact of the disclosed composition in/on the bleeding site. Methods for determining a level of appearance of bleeding are known in the art.

Further, in some embodiments, the terms "controlling", "preventing" or "reducing", in the context of the bleeding are also meant to encompass at least partially sealing blood vessels at the bleeding site e.g., in soft tissues.

In those instances where a convention analogous to "at least one of A, B, and C, etc." is used, in general such a construction is intended in the sense one having skill in the art would understand the convention (e.g., "a composition comprising at least one of A, B, and C" would include but not be limited to compositions that have A alone, B alone, C alone, A and B together, A and C together, B and C together, and/or A, B, and C together, etc.). It will be further understood by those within the art that virtually any disjunctive word and/or phrase presenting two or more alternative terms, whether in the description, claims, or drawings, should be understood to contemplate the possibilities of including one of the terms, either of the terms, or both terms. For example, the phrase "A or B" will be understood to include the possibilities of "A" or "B" or "A and B."

It is appreciated that certain features of the invention, which are, for clarity, described in the context of separate embodiments, may also be provided in combination in a single embodiment.

Various embodiments and aspects of the present invention as delineated hereinabove and as claimed in the claims section below find experimental support in the following non-limiting examples.

EXAMPLES

EXAMPLE 1: CHARECTERIZING THE FLOWABILITY PROPERTY OF SELECTED SAMPLES OF FIBRINOGEN THROMBIN BLENDS DISPERSED IN VARIOUS MEDIA

Properties like flowability, and gelation time need to be considered as a fundamental feature for flowable hemostat compositions.

In exemplary procedures, experiments were carried out to find the effect of selected dispersing media on the pasty consistency of compositions comprising powdered blend of fibrinogen and thrombin, without compromising the activity of the blend as hemostat.

In exemplary procedures made for the flowability tests, the powdered blend s comprised fibrinogen (88.23 %, by weight) thrombin (8.81 %, by weight) and $CaCl_2$ (2.94 %, by weight). Each blend was dispersed in a specific medium (also referred to herein as: "dispersant") at various weight ratios of blend-to-medium: 1/1.1=0.91:1, 1/1.3=0.77:1, 1/1.7=0.59:1, and 1/2=0.50:1 (Table 1).

The compositions of fibrinogen and thrombin were prepared by spray drying and were then combined with the medium. In some additional exemplary procedures, when indicated below as "HSMP" (high shear mixed powder) or "granulate", before the combination, an additional process was carried out to create granulated powders at certain particle size range (<106 or 106-250 $\mu$m) using high shear mix.

The media were selected from two categories: i) hydrophobic (specifically: soybean oil, "SO", from Aladdin CAS no. 8001-22-7; olive oil, "OO" from Aladdin CAS no. 8001-25-0; and mineral oil, "MO" from Aladdin CAS no. 8020-83-5); and ii) hydrophilic (specifically: propanediol, glycerol, and polyethylene glycol (PEG) 400).

In exemplary procedures, the particle size of the powdered fibrinogen thrombin was below 106 $\mu$m (using the high shear process) aimed to increase the homogeneity of the formulation

The flowability of selected samples was visually tested.

Table 1 presents detail444s on samples taken for the flowability tests

**Table 1 Samples for the Flowability Tests**

| # | Sample Name* | Fibrinogen thrombin powder (FTP) | | | | Medium |
|---|---|---|---|---|---|---|
| | | Fibrinogen % | Thrombin % | $CaCl_2$ % | Particle Size ($\mu$m) | |
| 1 | HSMP-SO | 88.23 | 8.81 | 2.94 | <106 | soybean oil |
| 2 | HSMP-OO | 88.23 | 8.81 | 2.94 | <106 | olive oil |
| 3 | HSMP-MO | 88.23 | 8.81 | 2.94 | <106 | mineral oil |
| 4 | HSMP-propanediol | 88.23 | 8.81 | 2.94 | <106 | propanediol |
| 5 | HSMP-glycerol | 88.23 | 8.81 | 2.94 | <106 | glycerol |
| 6 | HSMP-PEG400 | 88.23 | 8.81 | 2.94 | <106 | PEG400 |

*Batch#: XW20190410-01 (Bioseal, Guangzhou, Ethicon, US); samples were prepared about 3 months afterthe FTP production, by weighing 0.5g FTP to a container and adding 0.55/0.65/0.85/1g indicated dispersants to it, followed manually agitating for 5 min.

The results are shown in **Figure 1A** (pictures were taken within a few seconds after incorporating the blend in the indicated oil).

The results show that the oil media (SS, OO, MO) provided a flowable composition at the weight ratio of blend-to oil of 0.59:1 to 0.77:1. Higher ratios, such as 0.83, or 0.91 and above barely provided the desired flowability. Hydrophilic media- propanediol and glycerol, did not provide a flowable composition at the tested ratios.

A viscosity study of different powder to oil ratio, show (for soybean oil; **Figure 1B)** that the viscosity of the samples of the blend-to oil of 0.59 ratio to 0.77 ratio approaches 10 Pa·s. At the static stage (point 0), an appropriate static viscosity is between 10-100 Pa·s, the higher the viscosity at static stage, the more stable the paste is. The rapid dropping down of the curve with the 0.77 ratio means that the paste could be squeezed out of the syringe by an acceptable force (by hand). Thus, the upper limit is 0.91 (the composition being too dry, hard to be squeezed out), and the lower limit is 0.5 (the composition being too thin, not stable).

Although the hydrophilic medium, PEG400, provided a flowable composition at the weight ratios of blend-to oil of 0.59:1 and 0.50:1, it was found in additional experimental tests, that compositions comprising PEG400 barely formed a clot in the presence of saline as the PEG400 composition failed to form intact layer during the gelation test, which raised the concern of its hemostatic efficacy. **Figure 1C** presents illustrative comparative photographic images showing that when a hydrophobic dispersant (soybean oil) is used the paste forms an intact layer, curing with water after 7 min, whereas for PEG400 the paste failed to form an intact layer curing with water, after the indicated time.

In additional experiments, it was found that in the samples comprising oils at the corresponding ratio of below 0.59:1, the sample was too thin, and composition lost much of the hemostatic activity, as measured by clotting time. That is, higher flowability resulted in composition not capable of staying at wound site, and in slower gelation rate.

EXAMPLE 2: CHARECTERIZATION OF THE GELATION PROPERTY OF SELECTED SAMPLES - TESTING THE EFFECTS OF: ORC, POWDER PARTICLE SIZES AND OIL RATIOS

As described in Example 1 above, the oil was found to be suitable medium in terms of flowability.

In further exemplary procedures, compositions comprising the fibrinogen thrombin blends dispersed in oil were further tested to evaluate the effect of further factors: the particle size, the blend-to-oil ratio, and the presence of ORC which may serve as a wetting agent.

In exemplary procedures, the gelation property was tested on selected samples as listed in Table 2 below using soybean oil. Design was created by DOE in Minitab 18 which covers 3 factors ("2 levels").

**Table 2 Tested Samples and Main Properties (soybean oil was used; the powder was high shear mixed)**

| Test paste | Powder | | | | | Powder particle size (μm) | Oil ratio (powder to oil dispersant; w/w) |
|---|---|---|---|---|---|---|---|
| | Fibrinogen (%) | Thrombin (%) | CaCl$_2$ (%) | ORC (%) | Lysine* (%) | | |
| 1 | 68.90 | 6.88 | 2.36 | 15.62 | 6.25 | <106 | 1/1.4 =0.71 |
| 2 | 68.90 | 6.88 | 2.36 | 15.62 | 6.25 | <106 | 1/1.7=0.59 |
| 3 | 68.90 | 6.88 | 2.36 | 15.62 | 6.25 | 106-250 | 1/1.4=0.71 |
| 4 | 68.90 | 6.88 | 2.36 | 15.62 | 6.25 | 106-250 | 1/1.7=0.59 |
| 5 | 88.27 | 8.70 | 3.03 | 0.00 | 0.00 | <106 | 1/1.4=0.71 |
| 6 | 88.27 | 8.70 | 3.03 | 0.00 | 0.00 | <106 | 1/1.7=0.59 |
| 7 | 88.27 | 8.70 | 3.03 | 0.00 | 0.00 | 106-250 | 1/1.4=0.71 |
| 8 | 88.27 | 8.70 | 3.03 | 0.00 | 0.00 | 106-250 | 1/1.7=0.59 |
| *Lysine was added to neutralize the ORC acidity to maintain a neutral system for enzyme reaction | | | | | | | |

An exemplary experimental procedure for testing the gelation time was as follows:

a. Weighed 0.3 g paste was put on the steel plate evenly; the surface of paste was about 5 cm$^2$.

b. 0.3 ml saline was sprayed above the paste, followed by incubation at 37 °C and time was counted.

Gelation time was determined when gel was formed and could be peeled as an intact layer.

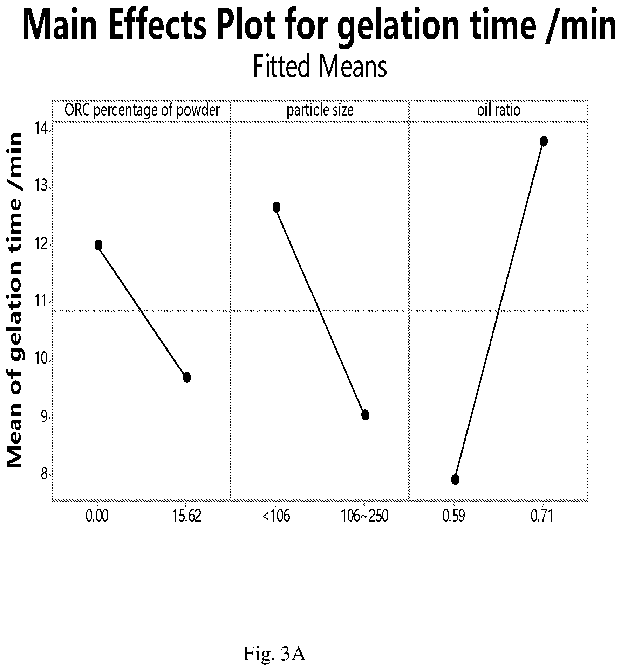

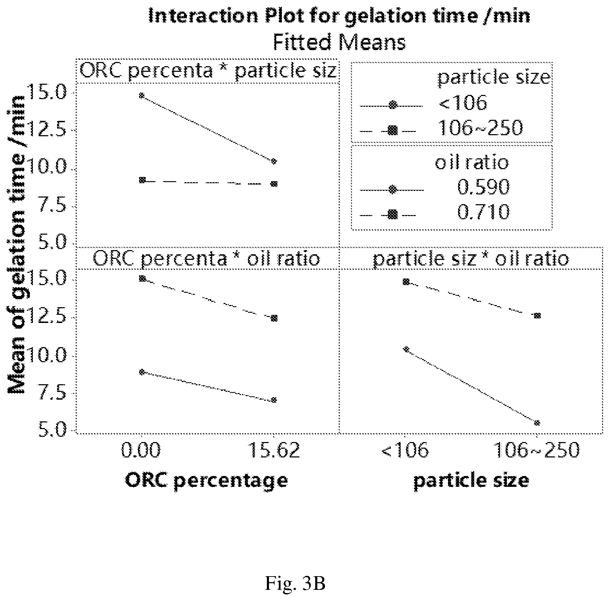

**Figure 2** shows average gelation time of each of group 1-8 as indicated in Table 2 (N=3). ANOVA was performed to analyze main effects among ORC concentration, powder particle size and oil ratio (see Table 3 below and graphic illustration of Table 3 in **Figure 3A** showing the Analysis of Variance for Gelation Time). The results suggest that all three factors had main effects on gelation time, with the powder-to-oil ratio contributing the main effect (ranked as "52.38 %" according to the linear model) and particles sized in the range of 106-250 micron provide a quicker gelation time. In addition, the combined interaction of ORC ratio and powder particle size was significant (ranked as "6.37 % contribution"), indicating the extent of correlation between gelation time and powder particle size depended on the ORC concentration (see illustration in **Figure 3B**).

**Table 3 Analysis of Variance for Gelation Time**

| Source | DF | Seq SS | Contribution | Adj SS | Adj MS | F-Value | P-Value |
|---|---|---|---|---|---|---|---|
| Model | 7 | 355.497 | 89.63 % | 355.497 | 50.785 | 19.77 | 0.000 |
| Linear | 3 | 316.436 | 79.79 % | 316.436 | 105.479 | 41.05 | 0.000 |
| **ORC ratio** | 1 | 31.259 | 7.88 % | 31.259 | 31.259 | 12.17 | 0.003 |
| **Powder particle size** | 1 | 77.436 | 19.52 % | 77.436 | 77.436 | 30.14 | 0.000 |
| **oil ratio** | 1 | 207.741 | 52.38 % | 207.741 | 207.741 | 80.85 | 0.000 |
| 2-Way Interactions | 3 | 36.711 | 9.26 % | 36.711 | 12.237 | 4.76 | 0.015 |
| ORC ratio*Powder particle size | 1 | 25.277 | 6.37 % | 25.277 | 25.277 | 9.84 | 0.006 |
| ORC ratio*oil ratio | 1 | 0.781 | 0.20 % | 0.781 | 0.781 | 0.30 | 0.589 |
| Powder particle size*oil ratio | 1 | 10.653 | 2.69 % | 10.653 | 10.653 | 4.15 | 0.059 |
| 3-Way Interactions | 1 | 2.350 | 0.59 % | 2.350 | 2.350 | 0.91 | 0.353 |
| ORC ratio*Powder particle size*oil ratio | 1 | 2.350 | 0.59 % | 2.350 | 2.350 | 0.91 | 0.353 |
| Error | 16 | 41.109 | 10.37 % | 41.109 | 2.569 | | |
| Total | 23 | 396.606 | 100.00 % | | | | |

Taken together, the experimental results show a positive effect of ORC on gelling speed. In addition, paste containing larger particles (106-250 $\mu$m) exhibited quicker gelation compared to that of smaller ones (<106 $\mu$m) (compare e.g., samples 1 and 7). Paste with higher oil dosage showed slower gelation.

However, in Example 3 below it will be shown that in terms of T-peel test which reflects paste adhesion, ORC negatively impacted T-peel force and no main effect was observed for oil dosage or particle size in this respect.

EXAMPLE 3: CHARECTERIZING THE EFFECT OF VARIOUS FACTORS ON THE ADHESIVE PROPERTY OF THE COMPOSITIONS

Adhesion plays a critical role for hemostat when applied to wound site for achieving efficient hemostasis. In order to screen fundamental factors of flowable fibrinogen thrombin paste, T-peel test was used to evaluate the impact of various factors including dispersing medium category (hydrophobic or hydrophilic), particle size of the blend, and the density of the blend of the composition on adhesion.

Exemplary procedures for T-peel test is based on ASTM F2256-05(2015) using a silicone sheet and includes:

a. Weighed 1g paste was applied on the surface of silicon sheet followed by saline spraying;
b. Another silicon sheet was put above the paste with 5 min compression by heavy weights; segment was cut to strips of 12 x 2 cm to fit the silicone sheets.
c. Prepared sheets were transferred to Instron testing system and the adhesion force was measured according to ASTM F2256-05(2015).
d. The force needed to separate the two adhered layers from one another (known as peel force) was measured using Instron 5944/100N sensor.

Sample preparation: FTP blend was made from porcine plasma. FTP comprised porcine fibrinogen, thrombin and in some tested samples further comprises ORC and lysine. Spraydraying, (and, when indicated, followed by high shear mixing (HSM)) was used to make FTP with higher (high sheared; 0.35 g/ml) or lower (0.25 g/ml) density. FTP was sieved to yield two categories of blends having different particle sizes (d<106$\mu$m, d=106-250 $\mu$m).

The protocol for spray draying is provided in Example 7 below.

***A first set of tested samples: Testing the effect of the medium (hydrophobic vs. hydrophilic) and the particle size:***

Testing samples having flowable property were made by mixing fibrinogen thrombin powdered blend ("FTP blend") with various media ("dispersants") at a fixed proportion (w/w=0.83). It is to note that although the 0.83 ratio barely provided flowability, the T-peel test has no requirement of sample flowability, and to meet the lowest detecting limit of the equipment, the samples were tested at this ratio so as to evaluate the T-peel force at a higher yield.

For this purpose, each silicone sheet was damped by saline followed by application of 1g of composition on the saline. Another silicone sheet was used to cover the sample after spraying saline again on the sample. A steel mold was put upon the sheets to press it for 5 min until curing took place.

Prepared sheets were transferred to Instron to measure the adhesion by T-peel test described above. Disclosed are results on average, minimum and maximum peel force and peel strength values. Average load per width was recorded and analyzed.

Information of tested samples are listed below.

HSMP250/MO: high shear mixing made FTP (d=106-250 $\mu$m) mixed with mineral oil.
HSMP/MO: high shear mixing made FTP (d<106 $\mu$m) mixed with mineral oil.
SP/MO: Spraying made FTP (d<106 $\mu$m) mixed with mineral oil.
HSMP/PEG400: high shear mixing made FTP (d<106 $\mu$m) mixed with PEG400.
HSMP/glycerol: high shear mixing made FTP (d<106 $\mu$m) mixed with glycerol.
HSMP/CO: high shear mixing made FTP (d<106 $\mu$m) mixed with canola oil.
HSMP/propanediol: high shear mixing made FTP (d<106 $\mu$m) mixed with propanediol.

**Results**

Table 4 shows the results of average load per width for each sample listed above; (sample size=3). As noted above, although glycerol and propanediol samples were found barely flowable, since the T-peel test method does not have strict requirement of flowability, they could be tested for providing additional references.

**Table 4 Average Load per Width**

| Sample | Mean (N/m) | STDEV | Minimum (N/m) | Maximum (N/m) |
|---|---|---|---|---|
| HSMP250/MO | 3.704 | 0.038 | 3.666 | 3.741 |
| HSMP/MO | 5.076 | 0.791 | 4.374 | 5.933 |
| SP/MO | 5.480 | 0.357 | 5.071 | 5.728 |
| HSMP/PEG400 | 1.934 | 0.262 | 1.632 | 2.097 |
| HSMP/glycerol | 4.261 | 0.510 | 3.702 | 4.7 |
| HSMP/CO | 3.141 | 0.828 | 2.195 | 3.738 |
| HSMP/propanediol | 2.032 | 0.371 | 1.684 | 2.423 |

One-way ANOVA was performed to analyze the difference among groups after confirming the pass of normality test and equal variances test (Table 5). The results showed that SP/MO achieved the highest adhesive property compared to other paste formulations (see **Figure 4**).

**Table 5 Tukey Simultaneous Tests for Differences of Means**

| Difference of Levels | Difference of Means | SE of Difference | 95% CI | T-Value | Adjusted P-Value |
|---|---|---|---|---|---|
| HSMP/PEG400 - HSMP250/MO | -1.770 | 0.426 | (-3.225, -0.315) | -4.15 | 0.013 |
| HSMP/glycerol - HSMP250/MO | 0.557 | 0.426 | (-0.898, 2.013) | 1.31 | 0.838 |
| HSMP/CO - HSMP250/MO | -0.563 | 0.426 | (-2.018, 0.892) | -1.32 | 0.832 |
| HSMP/MO - HSMP250/MO | 1.372 | 0.426 | (-0.083, 2.828) | 3.22 | 0.070 |
| SP/MO - HSMP250/MO | 1.776 | 0.426 | (0.321, 3.232) | 4.17 | 0.013 |
| HSMP/propane - HSMP250/MO | -1.672 | 0.426 | (-3.127, -0.216) | -3.92 | 0.020 |
| HSMP/glycerol - HSMP/PEG400 | 2.327 | 0.426 | (0.872, 3.783) | 5.46 | 0.001 |

(continued)

| Difference of Levels | Difference of Means | SE of Difference | 95% CI | T-Value | Adjusted P-Value |
|---|---|---|---|---|---|
| HSMP/CO - HSMP/PEG400 | 1.207 | 0.426 | (-0.248, 2.662) | 2.83 | 0.136 |
| HSMP/MO - HSMP/PEG400 | 3.142 | 0.426 | (1.687, 4.598) | 7.37 | 0.000 |
| SP/MO - HSMP/PEG400 | 3.546 | 0.426 | (2.091, 5.002) | 8.32 | 0.000 |
| HSMP/propane - HSMP/PEG400 | 0.098 | 0.426 | (-1.357, 1.554) | 0.23 | 1.000 |
| HSMP/CO - HSMP/glycerol | -1.120 | 0.426 | (-2.576, 0.335) | -2.63 | 0.189 |
| HSMP/MO - HSMP/glycerol | 0.815 | 0.426 | (-0.640, 2.270) | 1.91 | 0.504 |
| SP/MO - HSMP/glycerol | 1.219 | 0.426 | (-0.236, 2.674) | 2.86 | 0.130 |
| HSMP/propane - HSMP/glycerol | -2.229 | 0.426 | (-3.684, -0.774) | -5.23 | 0.002 |
| HSMP/MO - HSMP/CO | 1.935 | 0.426 | (0.480, 3.391) | 4.54 | 0.006 |
| SP/MO - HSMP/CO | 2.339 | 0.426 | (0.884, 3.795) | 5.49 | 0.001 |
| HSMP/propane - HSMP/CO | -1.109 | 0.426 | (-2.564, 0.347) | -2.60 | 0.197 |
| SP/MO - HSMP/MO | 0.404 | 0.426 | (-1.051,1.859) | 0.95 | 0.957 |
| HSMP/propane - HSMP/MO | -3.044 | 0.426 | (-4.499, -1.589) | -7.14 | 0.000 |
| HSMP/propane - SP/MO | -3.448 | 0.426 | (-4.903, -1.993) | -8.09 | 0.000 |

*Individual confidence level = 99.58%

**_The effect of the medium:_** The results (Table 4) show that those formulations which are SP/MO, HSMP/MO, HSMP/CO and HSMP/glycerol exhibit distinctly higher adhesive property compared to other formulations measured in this study.

Table 5 provides further indication of the differences between pairs of tested samples.

For example, the difference of the adhesiveness as tested by the "T-Peel test" between HSMP/MO to HSMP/PEG400 was 3.142 N/m (according to the mean values presented in Table 4, i.e. 5.076-1.934), indicating that oils, such as mineral oil, provide stronger adhesive property as compared to hydrophilic medium such as PEG400.

It is noteworthy that a composition of hydrophilic media, i.e. glycerol, showed a relative strong adhesiveness (4.261 N/m), however, again, such hydrophobic media barely met the requirement of flowability as shown in Example 1 above, and is therefore not preferred.

Without being bound by any particular theory, it may be conceivable that paste formulation containing oils such as mineral oil displays better adhesion since the fibrinogen remains active in such media.

**_The effect of the particle size_**: it can also be concluded that the particles size significantly affected adhesion, as determined based on Table 5 above (compare, for example, HSM/MO with HSM250/MO). As will be shown in Example 4 below, paste with larger granules typically exhibited poor homogeneity, perhaps since the paste formulation comprises two separated phases: solid particles and liquid hydrophobic media. Without being bound by any mechanism, smaller granules may mitigate the separation of phases thus improving homogeneity and the final outcome.

**_A second set of tested samples- testing the effect of ORC and additional ingredients:_**

In a further study, further formulations were developed, focusing on hydrophobic dispersant, mineral oil for instance, as well as finer blend of fibrinogen thrombin powder(FTP).

In exemplary procedures, the following samples of blends composed of FTP blend were prepared for the adhesiveness tests (see Table 6 for further details):

#1 HSMP-ORCF-SP: fibrinogen and thrombin small powder without ORC (d<106 $\mu$m);
#2 HSMP-ORCF-LP: fibrinogen and thrombin large powder without ORC (d=106-250 $\mu$m);
#3 HSMP-ORC 15-SP: fibrinogen and thrombin small powder (d < 106 $\mu$m) containing about 15 % ORC and 6.24 % lysine.

**Table 6 FTP Blends**

| # | Fibrinogen % | Thrombin % | CaCl$_2$ % | ORC % | lysine % | particle size ($\mu$m) | D10 ($\mu$m) | D50 ($\mu$m) | D90 ($\mu$m) |
|---|---|---|---|---|---|---|---|---|---|
| 1 | 88.23 | 8.81 | 2.94 | 0 | 0 | <106 | 23.36 | 47.22 | 82.75 |
| 2 | 88.23 | 8.81 | 2.94 | 0 | 0 | 106-250 | 32.86 | 133.9 | 293 |
| 3 | 68.94 | 6.89 | 2.3 | 15.63 | 6.24 | <106 | 27.74 | 58.6 | 100.8 |

FTP blend was made using porcine plasma according to the procedures described below (spray drying followed by high shearing); FTP blend comprising porcine fibrinogen and thrombin were sieved to yield two particle sizes (d<106 $\mu$m, d=106-250 $\mu$m). FTP containing 15 % ORC fiber (80 $\mu$m) and 6.24 % lysine was further tested.

For the flowable composition, preparation of FTP blend was mixed with the indicated dispersing medium to form a flowable composition. As shown in Example 1 above, suitable ratio of medium to FTP blend was determined by composition appearance which must be moderate flowable. Thus, fixed ratio of powder to dispersant 0.59 was chosen in view of the flowability tests presented in Example 1. Below (Table 7) is shown information of tested compositions for this set of exemplary procedures, including any one of the above #1-3 blends (referred to as "FTP #" in Table 7) and further combined with media, forming a pasty consistency.

**Table 7 Sample tested**

| Sample# | Sample name | FTP # | Medium | FTP to medium (w/w) |
|---|---|---|---|---|
| 1 | HSMP-ORCF-SP-SO | 1 | soybean oil | 0.59 |
| 2 | HSMP-ORCF-SP-OO | 1 | olive oil | 0.59 |
| 3 | HSMP-ORCF-SP-MO | 1 | mineral oil | 0.59 |
| 4 | HSMP-ORCF-SP-propanediol | 1 | propanediol | 0.59 |
| 5 | HSMP-ORCF-SP-glycerol | 1 | glycerol* | 0.59 |
| 6 | HSMP-ORCF-SP-PEG400 | 1 | PEG400 | 0.59 |
| 7 | HSMP-ORC15-SP-MO | 3 | mineral oil | 0.59 |
| 8 | HSMP-ORCF-LP-MO | 2 | mineral oil | 0.59 |
| *glycerol at 0.59 ratio had no pasty consistency. | | | | |

**Results**

Common results are presented below showing the average, minimum and maximum peel force and peel strength values. Average load per width was recorded and analyzed.

The average load per width for each sample are as follows (sample size=3) (Table 8):

**Table 8 Average Load per Width for each Sample**

| Sample# | Sample name | Mean (N/m) | STDEV | Minimum (N/m) | Maximum (N/m) |
|---|---|---|---|---|---|
| 1 | HSMP-ORCF-SP-SO | 2.814 | 0.208 | 2.574 | 2.951 |
| 2 | HSMP-ORCF-SP-OO | 3.048 | 0.369 | 2.690 | 3.427 |
| 3 | HSMP-ORCF-SP-MO | 3.612 | 0.374 | 3.369 | 4.043 |
| 4 | HSMP-ORCF-SP-propanediol | 2.263 | 0.263 | 2.048 | 2.556 |
| 5 | HSMP-ORCF-SP-glycerol | 3.428 | 0.183 | 3.280 | 3.632 |
| 6 | HSMP-ORCF-SP-PEG400 | 3.330 | 0.769 | 2.736 | 4.199 |
| 7 | HSMP-ORC15-SP-MO | 3.134 | 0.336 | 2.802 | 3.473 |
| 8 | HSMP-ORCF-LP-MO | 3.576 | 0.734 | 2.944 | 4.381 |

The results are further illustrated in **Figure 5.**

One-way ANOVA was performed to analyze the difference among groups after confirming the pass of normality test and equal variances test, as presented in Table 9.

**Table 9 Analysis of Variance**

| Source | DF | Adj SS | Adj MS | F-Value | P-Value |
|--------|----|--------|--------|---------|---------|
| Sample | 7 | 4.244 | 0.6063 | 2.91 | 0.036 |
| Error | 16 | 3.331 | 0.2082 | | |
| Total | 23 | 7.575 | | | |

The analysis of variance shows that significant difference was observed among groups ($p$=0.036). Tukey comparisons for the means of each respective test were indicated and are presented in Table 10.

**Table 10 Grouping Information Using the Tukey Method and 95 % Confidence**

| Sample | N | Mean | *Grouping | |
|--------|---|------|-----------|---|
| HSMP-ORCF-MO | 3 | 3.612 | A | |
| HSMP-ORCF-LP-MO | 3 | 3.576 | A | |
| HSMP-ORCF-glycerol | 3 | 3.428 | A | B |
| HSMP-ORCF-PEG400 | 3 | 3.330 | A | B |
| HSMP-ORC 1/5-MO | 3 | 3.134 | A | B |
| HSMP-ORCF-OO | 3 | 3.048 | A | B |
| HSMP-ORCF-SO | 3 | 2.814 | A | B |
| HSMP-ORCF-propanediol | 3 | 2.263 | | B |

*Means that do not share a letter are significantly different.

In Table 10 means that do not share the same letter are statistically significant. For example, the results suggest that formulations of HSMP-ORCF-MO and HSMP-ORCF-LP-MO (both are signed by letter "A" only) exhibit distinctly higher adhesive property according to highest T-peel force compared to that of HSMP-ORCF-propanediol (signed by letter "B" only), indicating mineral oil is a desired medium.

It also appears that the addition of ORC decreases the T-peel force (compare samples 3 and 7 in Table 8).

No significant different was observed among other compositions of glycerol, PEG400, olive oil, soybean oil in terms of adhesion. However, as provided in Example 1, other properties like flowability, as well as gelation time, need to be considered as fundamental features for this concept. As presented above, composition of glycerol, for instance, showed high adhesive property but barely meet the requirement of flowability.

***A third set of tested samples - testing the combined effects of ORC, particle size and oil ratio:***

The tested samples are provided in Table 11 (which is Table 2 being copied hereto for convenience).

**Table 11 Tested Samples for the Third Set of Tested Sampling**

| Test paste | Powder | | | | | Powder particle size (μm) | Oil ratio (powder to oil dispersant; w/w) |
|---|---|---|---|---|---|---|---|
| | Fibrinogen (%) | Thrombin (%) | CaCl$_2$ (%) | ORC (%) | Lysine (%) | | |
| 1 | 68.90 | 6.88 | 2.36 | 15.62 | 6.25 | <106 | 1/1.4 =0.71 |
| 2 | 68.90 | 6.88 | 2.36 | 15.62 | 6.25 | <106 | 1/1.7=0.59 |
| 3 | 68.90 | 6.88 | 2.36 | 15.62 | 6.25 | 106-250 | 1/1.4=0.71 |
| 4 | 68.90 | 6.88 | 2.36 | 15.62 | 6.25 | 106-250 | 1/1.7=0.59 |
| 5 | 88.27 | 8.70 | 3.03 | 0.00 | 0.00 | <106 | 1/1.4=0.71 |
| 6 | 88.27 | 8.70 | 3.03 | 0.00 | 0.00 | <106 | 1/1.7=0.59 |
| 7 | 88.27 | 8.70 | 3.03 | 0.00 | 0.00 | 106-250 | 1/1.4=0.71 |

(continued)

| Test paste | Powder | | | | | Powder particle size (μm) | Oil ratio (powder to oil dispersant; w/w) |
| | Fibrinogen (%) | Thrombin (%) | CaCl₂ (%) | ORC (%) | Lysine (%) | | |
|---|---|---|---|---|---|---|---|
| 8 | 88.27 | 8.70 | 3.03 | 0.00 | 0.00 | 106-250 | 1/1.7=0.59 |

The T-peel test was conducted to evaluate adhesive property for fibrinogen paste listed in Table 11. Statistical analysis showed that the ORC ratio was a main factor regarding to promoting adhesion (as presented in Table 12 below; 22.53 % contribution in the linear model).

**Table 12 T-Peel Test Statistics**

| Source | DF | Seq SS | Contribution | Adj SS | Adj MS | F-Value | P-Value |
|---|---|---|---|---|---|---|---|
| **Model** | 7 | 12.4975 | 82.88% | 12.4975 | 1.78536 | 11.06 | 0.000 |
| Linear | 3 | 3.8235 | 25.36% | 3.8235 | 1.27452 | 7.90 | 0.002 |
| **ORC ratio** | 1 | 3.3968 | 22.53% | 3.3968 | 3.39679 | 21.05 | 0.000 |
| particle size | 1 | 0.2964 | 1.97% | 0.2964 | 0.29637 | 1.84 | 0.194 |
| oil ratio | 1 | 0.1304 | 0.86% | 0.1304 | 0.13039 | 0.81 | 0.382 |
| 2-Way Interactions | 3 | 8.6475 | 57.35% | 8.6475 | 2.88250 | 17.86 | 0.000 |
| ORC ratio*particle size | 1 | 4.0846 | 27.09% | 4.0846 | 4.08458 | 25.31 | 0.000 |
| ORC ratio*oil ratio | 1 | 0.3302 | 2.19% | 0.3302 | 0.33018 | 2.05 | 0.172 |
| particle size*oil ratio | 1 | 4.2328 | 28.07% | 4.2328 | 4.23276 | 26.23 | 0.000 |
| 3-Way Interactions | 1 | 0.0265 | 0.18% | 0.0265 | 0.02647 | 0.16 | 0.691 |
| ORC ratio*particle size*oil ratio | 1 | 0.0265 | 0.18% | 0.0265 | 0.02647 | 0.16 | 0.691 |
| Error | 16 | 2.5821 | 17.12% | 2.5821 | 0.16138 | | |
| Total | 23 | 15.0797 | 100.00% | | | | |

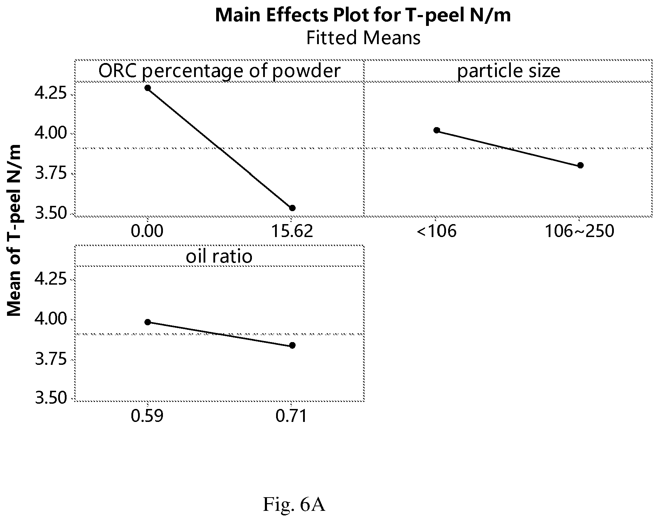

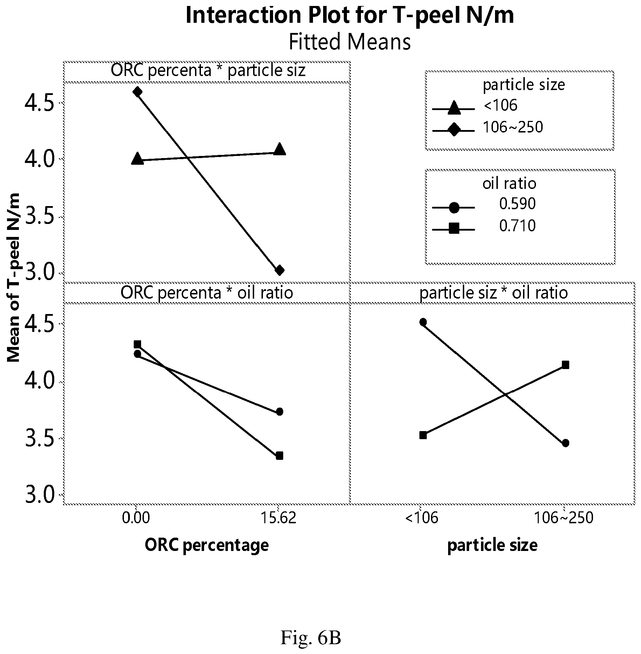

The results show that contradicting to its positive role for gelation process (see above), the addition of ORC decreased the T-peel force (see **Figure 6A**). This effect also related to particle size as the combined interaction of ORC ratio and particle size was significant. No main effect was observed for particle size or oil ratio itself due to their antagonistic interaction (see **Figure 6B**). However, in the next Examples it is shown that formulations based on small particles size range such as less than 106 micron provide better homogeneity.

EXAMPLE 4: HOMOGENEITY AND STABILITY TESTS

*Formulation homogeneity:* the protocol provides a method to determine the homogeneity of the paste formulation by evaluating remnant within applicator after expression force applied.
Equipment: Instron 5944/1kN sensor; Fix holder, and Electric caliper.
in exemplary procedures, formulation homogeneity was examined on the formulations described in Table 13 below.

**Table 13 Test Groups for Homogeneity Examination (Groups B and C are high sheared mixed)**

| Category | Fibrinogen % | Thrombin % | CaCl₂ % | ORC % | Lysine % | Particle Size (μm) | D10 (μm) | D50 (μm) | D90 (μm) | SO ratio |
|---|---|---|---|---|---|---|---|---|---|---|
| A | 88.23 | 8.81 | 2.94 | 0 | 0 | - | 9.71 | 30.30 | 51.92 | 0.59 |
| B(granular) | 88.23 | 8.81 | 2.94 | 0 | 0 | <106 | 23.36 | 47.22 | 82.75 | 0.59 |
| C(granular) | 88.23 | 8.81 | 2.94 | 0 | 0 | <250 | 16.31 | 47.35 | 151.3 | 0.59 |

Formulation inhomogeneity can be determined based on the observation of liquid/solid phase separation which causes remnant within applicator (syringe) after a fixed pressing force applied.
In exemplary procedures, 3 g anhydrous paste was filled into a 3 ml syringe (applicator). The height of the paste volume in syringe was measured as "H" *via* electric caliper. Next, the filled syringe was transferred to the fixed holder, while ensuring that the syringe was set vertically. An upper limit of expression force was set 80N in the applicator. Expression ceased once reaching to 80 N, and remnant within the syringe was weighed.

Accordingly, the testing program was set with a maximum load of 80 N in a speed of 3.33 mm/s. The sensor was adjusted to approach the plunger and to ensure that the initial load was in the range of ± 0.03 N. The Expression force was applied to the filled syringe until it reached to the maximum load, and the displacement was recorded as "D".

The remnant concentration was calculated *via* the following equation:

$$\text{Remnant} = [(H-D)/H] \times 100\ \%.$$

Criterium for acceptable homogeneity is the remnant being less than 5%.

The results are present in **Figure 7**, showing that with formula comprising particles' size in the range of 16 to 151 micron 4.9 % (by weight) remnant was detected; with formula comprising particles' size in the range of 23-83 micron 3.4 % (by weight) remnant was detected; and no remnant was observed in formula comprising particles' size in the range of 10-52 micron. Hence, the results show that compositions comprising smaller particles had fewer remnant in syringe after pressing, suggesting that smaller particles (below 106 micron) may achieve better homogeneity.

*Stability:* in exemplary procedures, formulation stability was examined on the formulations described in Table 14 below.

**Table 14 Test Groups for Stability Examination (Groups B is high sheared mixed)**

| Category | Fibrinogen % | Thrombin % | CaCl$_2$ % | ORC % | Lysine % | Particle Size ($\mu$m) | D10 ($\mu$m) | D50 ($\mu$m) | D90 ($\mu$m) | SO ratio |
|---|---|---|---|---|---|---|---|---|---|---|
| A | 88.23 | 8.81 | 2.94 | 0 | 0 | - | 9.71 | 30.30 | 51.92 | 0.59 |
| B(granular) | 88.23 | 8.81 | 2.94 | 0 | 0 | <106 | 23.36 | 47.22 | 82.75 | 0.59 |

*Formulation stability:* in exemplary procedures, an upper limit of expression force was set 80N in the applicator. Exemplary procedures were carried out as described above for the homogeneity tests, with remnant within the syringe being evaluated at 4 time points: 0, 7, 23, 30 days at room temperature.

The results are presented in **Figure 8,** showing that with formula comprising larger particles' size, about 20 % (by weight) remnant was detected after 23 days; formula comprising smaller particles' size only 4 % (by weight) remnant was detected (after 30 days); Hence, the results suggest that smaller particles may achieve a better stability.

EXAMPLE 5: USING COLLAGEN FOR IMPROVING RHEOLOGICAL PROPERTIES

In additional exemplary procedures, another set of experiments were carried out to further investigate and improve the rheological properties of tested samples (1)-(5) by adding thereto collagen (purchased from Shanghai D&B Biological Science and Technology Co. Ltd.).

The oil used was soybean oil.

The samples (ratios refer to powder-to-oil ratios by weight; percent of collagen is by total weight including the oil, no ORC):

(1) 1:1.7, no collagen;
(2) 1:1.7, 10 % collagen;
(3) 1:1.7, 20 % collagen;
(4) 1:1.5, 10 % collagen; and
(5) Gelatin matrix (SURGIFLO®).

*Amplitude Sweep Test:* In an exemplary set of experiments, shear loss (G') and shear storage (G") modulus of the samples were evaluated via shear rheometer. The measurements were conducted using Anton Paar Modular Compact Rheometer MCR102. All tests were conducted at a temperature of 25 °C. A strain sweep tests were conducted to determine the linear viscoelastic regime of the samples. Sweep tests were then performed at a Shear Strain (oscillating) range of 0.01 to 100 %, angular frequency in the range of 010 rad/sec.

During an amplitude sweep the amplitude of the deformation - or alternatively the amplitude of the shear stress - is varied while the frequency is kept constant. The amplitude is the maximum of the oscillatory motion. For the analysis the storage modulus G' and the loss modulus G" are plotted against the deformation.

The intersection of G' and G" is the yield point ($\tau$). $\tau$ = F/A with shear stress $\tau$ (tau), shear force F (in N) and shear

area A (in $m^2$). The tau value for sample (1) is 0.1328 Pa, sample (2) is 0.7824 Pa, sample (3) is 4.501 Pa and sample (4) is 13.51 Pa, meaning that the force needed to push the sample go forward is directly proportional to the collagen concentration and is reversely proportional to powder-to-oil ratio. Compared to the control (Surgiflo) the yield point of each sample is smaller, indicating that the range 1:1.5 to 1:1.7, and 0-20 % collagen can result in a formulation with the appropriate pushing force during the application.

*Thixotropy (3ITT) Test:* in an additional set of exemplary experiments, three-interval thixotropy test was performed using Anton Paar Modular Compact Rheometer MCR102. The test was performed to evaluate the deformation and regeneration of different paste formulation. The results of the tests with three intervals were depicted as a time-dependent viscosity function: (I) at the beginning, high viscosity at rest (point every 6 sec for total interval of 60 sec; shear rate: constant, 1/s), (II) decrease in viscosity caused by structural breakdown induced by high shear (point every 5 sec for total interval of 5 sec; shear rate: constant, 100/s), (III) increase in viscosity by structural recovery at rest (point every 2 sec for total interval of 200 sec; shear rate: constant, 1/s). The results are shown in **Figure 9.** It can be seen that the sample without collagen exhibited very slow structural recovery, indicating that the sample will run away once applied on the wound site. According to the 3ITT result, the powder to oil ratio is 1.5 to 1.7, and the collagen % improves the viscosity behavior up to 20 %.

*Gelation Test:* in an additional set of exemplary experiments the gelling time was tested was performed using Anton Paar Modular Compact Rheometer MCR102. 5 ml of water was added to each sample. The results of the tests were collected (300 datapoints every 2 sec; shear strain oscillating 5 %; constant frequency of 10Hz). The intervals between the water adding point and the crossing point (G'=G") represents the gelling time of each tested samples. The results show that for samples (1) and (2) the gelling time was less than 3 min. The crossing point of sample (3) and (4) could not be seen within 10 min, indicating these two formulations have a long gelling time, and may be less optimal for hemostatic applications.

Taken together, it can be concluded that the addition of collagen, up to 20 % by weight (optimally abount 10 %), the water absorption property of the paste is significantly improved, which might further impact the hemostasis time in practical applications.

EXAMPLE 6: *IN VIVO* TESTS

In exemplary procedures, the following samples were tested on spleen biopsy punch model under heparinized condition. **Figure 10A** presents a typical flow chart showing the usage of the sample on a biopsy punch bleeding model.

In exemplary procedures, the following samples (Table 15) were tested.

**Table 15** **Test Groups for *in vivo* Examination (Groups 2-4 are high sheared mixed**)

| # | Fibrinogen % | Thrombin % | $CaCl_2$ % | ORC % | Lysine % | Particle Size ($\mu$m) | D10 ($\mu$m) | D50 ($\mu$m) | D90 ($\mu$m) | SO ratio |
|---|---|---|---|---|---|---|---|---|---|---|
| 1 | 88.23 | 8.81 | 2.94 | 0 | 0 | - | 9.71 | 30.30 | 51.92 | 0.67 |
| 2 | 77.44 | 7.65 | 2.63 | 8.78 | 3.51 | <106 | 23.36 | 47.22 | 82.75 | 0.63 |
| 3 | 88.23 | 8.81 | 2.94 | 0 | 0 | <106 | 23.36 | 47.22 | 82.75 | 0.71 |
| 4 | 88.23 | 8.81 | 2.94 | 0 | 0 | <250 | 16.31 | 47.35 | 151.3 | 0.71 |

In exemplary procedures, a punch hole of 5 mm was made deep on the surface of the spleen with a 6 mm puncher. Prior to testing for article application, excess blood was blotted removed from the target bleeding site with gauze or by using suction so the hemostatic powders could be applied directly to the bleeding site with the bleeding surface as dry as possible before the product application. Sufficient amount of paste or powder was applied to the site to cover the entire bleeding area with multiple layers. A dry gauze was then applied immediately to hold the test article in contact with the bleeding area. Following 2 min of waiting the hemostasis efficacy was evaluated. Durable hemostasis was evaluated during a 1-minute observation period following irrigation of the site with 10 mL of saline.

Representative results for spleen biopsy punch model under heparinized condition are shown in **Figure 10B,** showing that the particle size did not have any impact on the hemostatic efficacy adhesion detection in a bleeding site upon injection of 1 ml of formulation and after 1 min tamponade in the surgical site.

In conclusion, within the range of flowability, lower oil dosage and larger particle size allow to achieve faster gelation. However, small sized particles may be preferred since paste with the larger granules typically exhibited poor homogeneity and stability. For ORC addition, a moderate level within those tested should be selected, if at all, to balance the need of gelation speed and adhesive property. Collagen at certain concentrations may improve the rheological properties.

EXAMPLE 7: THE PRODUCTION PROCESSES OF THROMBIN AND FIBRINOGEN POWDERS BY THE SPRAY DRYING METHOD

***Raw materials:***

Vendors: Bioseal, Guangzhou, Ethicon, US.

Source of thrombin/Fibrinogen: porcine plasma;
Amount of Fibrinogen in the blend depends on ORC additive as follows: ORC=0, FIB%=88.23; ORC%=8.78, FIB%=77.44; ORC%=15.63, FIB=68.94: Remaining includes thrombin, $CaCl_2$, Lysine. Reference is made to Table 16 below.

ORC (used in some samples), in amount of 8.79 %, by weight; ORC characteristic: particle size <70 $\mu$m, fiber form.

*Spray Drying:* spray drying is a drying method used to create powder from a solution, suspension or emulsion that is atomized through a spray nozzle in a hot airflow and dried instantly. Spray drying process is controlled by several process parameters, among them are: column air flow and temperatures, nozzle size and atomizing air flow rate, material flow rate, etc.

In exemplary procedures, thrombin and fibrinogen powders were produced using a spray drying method (Spray dryer: 4M8-TriX spray dryer was used (by ProCepT nv, Zelzate, Belgium) as follows.

Prepared thrombin and fibrinogen porcine fibrinogen solutions were drawn into a syringe and placed inside the syringe pump of the spray dryer. The syringe pump was set to the desired flow rate with feed valve closed. While the syringe pump feed valve was closed, the spray dryer was activated and the desired atomizing gas flow rate, drying gas flow rate, drying gas temperature, cooling gas flow rate, and cyclone gas flow rate were set.

The cooling gas flow rate and temperature were selected such as not to disrupt laminar flow in the drying column, but to reduce the gas flow temperature to below the glass transition temperature of the composition in order to prevent powder from sticking to the glass parts.

The spray dryer was allowed to run until a steady state was reached where the actually measured value of the parameters reached the set levels and remained steady. The feed valve was then opened, allowing the thrombin/fibrinogen solutions to flow through the feed inlet to the spray nozzle to be atomized by the atomizing gas flow to small droplets which then dried in the drying column. Spray dried powder was formed, which was collected in the powder outlet of the cyclone of the spray dryer.

The spray dried powder recovered from the cyclone was weighed in a de-humidifier at a relative humidity of below 30 % and divided into samples of between 100 - 200 mg. Each sample was individually sealed in a test tube with a plug and sealed with Parafilm® (Bemis, Oshkosh, Wisconsin, USA) until evaluation.

Thrombin and fibrinogen maintained their: activity, low water content, high solid yield and desired powder particle size and distributions. The obtained particles were mixed, dispersant additive was added, and the obtained formulation was homogenized.

The main spray drying process parameter ranges are detailed below in Tables 16 and 17 below.

**Table 16 Spray Dry of Fibrinogen Formulation**

| Parameters | Value |
|---|---|
| Drying Columns | 3 (Mode II) |
| Column Air Flow | 0.6 $m^3$/min |
| Inlet Air Temperature | 150 °C |
| Nozzle Diameter | 0.8 mm |
| Atomizing Air Flow | 12 L/min |
| Cyclone Gas | 0.15 $m^3$/min |

**Table 17 Spray Dry of Thrombin Formulation**

| Parameters | Value |
|---|---|
| Drying Columns | 2 |
| Column Air Flow | 0.3 $m^3$/min |
| Cooling Air Flow | 0.3 $m^3$/min |
| Inlet Air Temperature | 160 °C |

(continued)

| Parameters | Value |
|---|---|
| Nozzle Diameter | 0.4 mm |
| Atomizing Air Flow | 7 L/min |
| Cyclone Gas | 0.1 m$^3$/min |

*High Shear:* in some exemplary procedures of making the powdered blend, the spry dry process was applied, and upon obtaining the fibrinogen and thrombin particles and prior to adding the dispersant, the particles were mixed and high sheared. This process relates to making powder using high shear mixer with 1L bowl at the scale of 100g material.

*Equipment details:* Balance: SS1000; High shear mixer: Mini-CG 1L; Syringe pump: LSP01-1C; Vacuum dry: Vacucell 111; Vibrating screen: Fritsch analysette 3.

Materials: Raw materials formulation (weight percentage): 1. With ORC: 77.44 % fibrinogen powder; 7.60 % thrombin powder; 2.64 % $CaCl_2$ powder; 8.79 % ORC powder, 3.52 % lysine powder. 2. Without ORC: 88.32 % fibrinogen powder; 8.67 % thrombin powder, 3.01 % $CaCl_2$ powder. Suspending agent: 3M Nonafluoromethoxybutane (HFE 7100). Binder: purified water.

*Environment:* Temperature: 22-26 °C; Relative Humidity: 50 %-70 %.

In exemplary procedures, a suspension was created in a high shear mixing/shearing reactor, having a 150-500 rpm speed mixing blade and 150-500 rpm cutting blade. Specifically, in the premixing step, the raw materials were weighed for a total weight of 100 g. The materials were put into the 1L bowl of high shear mixer. Chopper speed was set at 150 rpm, impeller speed was set at 150 rpm, mixing for 2 mins. Next, 325 ml HFE was put into the bowl to suspend the raw materials, mixing for 2 mins. Next, in the granulation step, the chopper speed was set at 200 rpm, impeller speed was set at 200 rpm, and the sealing pressure was set at 0.05 MPa. Using a syringe pump, 10 ml purified water was sprayed into the bowl to bind the particles of suspension, in a flowrate of 4 ml/min, and nozzle size 0.4 mm.

Atomization pressure was 0.05 MPa. Granulation time: 2 mins. Next, in the post-granulation step, the chopper speed was adjusted to 300 rpm, and the impeller speed to 500 rpm. Granulation was then continued for 2 minutes. In the granulation step, the HFE volatilized. It is to note that since HFE 7100 was used as a suspending agent, a water bath jacket was used to help the HFE 7100 volatilizing, therefore, the water temperature was set to 45 °C. At the end of this step, no liquid was visible.

Next, a first vacuum drying was applied: the obtained composition was transferred to a tray ant put into vacuum drying box. Vacuum pressure was set to approximately 0 Pa for 3 hours. Next, the powder was sieved: the powder was transferred to the vibrating screen tray, the sieve size was in proper order 250/106 micron. The amplitude was set at 1.5 mm, sieving time: 10 mins.

Next, a second vacuum drying was applied: the powder was transferred into the vacuum drying box, kept for drying for 3 hours, with the vacuum pressure being approximately 0 Pa. Finally, the product was collected below 250 $\mu$m sieves.

Exemplary data of particle size for "raw material" and granular powder obtained by the high shear process are provided in Table 18 below.

**Table 18 Particle Size for Raw Material and Granular Powder (For row #1 and #2, fibrinogen and thrombin powder are output of spray drying process without further high shearing. For the rest of rows (noted "granular"), the granular particles are output of high shear mixing process)**

| Category | Fibrinogen % | Thrombin % | CaCl$_2$ % | ORC % | Lysine % | Particle Size ($\mu$m) | D10 ($\mu$m) | D50 ($\mu$m) | D90 ($\mu$m) |
|---|---|---|---|---|---|---|---|---|---|
| Raw material | 100 | - | - | - | - | - | 9.71 | 30.30 | 51.92 |
| Raw material | | 100 | - | - | - | - | 7.597 | 18.17 | 34.72 |
| Granular powder | 88.23 | 8.81 | 2.94 | 0 | 0 | <106 | 23.36 | 47.22 | 82.75 |
| Granular powder | 88.23 | 8.81 | 2.94 | 0 | 0 | 106-250 | 32.86 | 133.9 | 293 |
| Granular powder | 88.23 | 8.81 | 2.94 | 0 | 0 | <250 | 16.31 | 47.35 | 151.3 |
| Granular powder | 68.94 | 6.89 | 2.3 | 15.63 | 6.24 | <106 | 27.74 | 58.6 | 100.8 |
| Granular powder | 68.94 | 6.89 | 2.3 | 15.63 | 6.24 | 106-250 | 49.15 | 141 | 328 |

**Claims**

1. A composition comprising: (i) a blend of fibrinogen and thrombin, wherein the blend is in the form of a plurality of particles, and (ii) a hydrophobic dispersant, wherein the composition is in the form of a paste at at-least one temperature in the range of 10 °C to 37 °C.

2. The composition of claim 1, wherein the paste further comprises collagen.

3. The composition of claim 2, wherein the collagen is present at a concentration of below 20 % by weight, optionally wherein the collagen is present at a concentration of at least about 10 % by weight.

4. The composition of any one of claims 1 to 3, wherein the paste is **characterized by** a yield point ranging from about 0.1 to about 15 Pa, as measured at a temperature of 25 °C and frequency of 10 rad/sec.

5. The composition of any one of claims 1 to 4, wherein the ratio of said blend to the hydrophobic dispersant ranges from 0.5:1 to less than 0.9:1 (w/w), respectively.

6. The composition of any one of claims 1 to 5, wherein the hydrophobic dispersant is selected from the group consisting of vegetable oil, mineral oil, and a combination thereof, preferably, wherein the hydrophobic dispersant comprises mineral oil.

7. The composition of any one of claims 1 to 6, wherein the hydrophobic dispersant comprises an oil selected from the group consisting of soybean oil, olive oil, cholesteryl oleate, corn oil, triolein, safflower oil, squalene, squalane, dodecane, and any mixture thereof.

8. The composition of any one of claims 1 to 7, wherein the fibrinogen is present at a concentration range of 60 % to 95 %, or 70 % to 90 %, by weight of the blend, preferably, wherein the fibrinogen is present at a concentration range of 60 % to 85 %, by weight of the blend, and the thrombin is present at a concentration range of 5 %, to 10 %, by weight of the blend.

9. The composition of any one of claims 1 to 8, wherein the blend comprises less than 6 % water, by weight.

10. The composition of any one of claims 1 to 9, comprising less than 3.5 % water, by total weight.

11. The composition of any one of claims 1 to 10, wherein the blend further comprises one or more excipients selected from the group consisting of: amino acids, albumin, saccharides, or any derivative or mixture thereof.

12. The composition of claim 1 to 11, further comprising a calcium salt and/or lysine.

13. The composition of any one of claims 1 to 12, wherein the plurality of particles is **characterized by** a median particles size of up to 270 μm, preferably,
wherein the plurality of particles is **characterized by** a median particles size of about 8 to about 60 μm.

14. The composition of any one of claims 1 to 13, comprising oxidized cellulose (OC) at a concentration of less than 12 %, e.g., 5 to less than 12 %, preferably, wherein the oxidized cellulose (OC) is oxidized regenerated cellulose (ORC).

15. The composition of any one of claims 1 to 13, being devoid of oxidized cellulose (OC), preferably, wherein the oxidized cellulose (OC) is oxidized regenerated cellulose (ORC).

16. The composition of any one of claims 1 to 15, wherein the blend is spray-dried mixed, and/or wherein the blend is high-shear mixed.

17. The composition of any one of claims 1 to 16, wherein the thrombin and/or fibrinogen originates from porcine plasma.

18. The composition of any one of claims 1 to 17, being a hemostatic composition.

19. The composition of any one of claims 1 to 18, for use in a method for treating a bleeding tissue.

20. The composition for use according to claim 19, wherein the composition is applied on a surface of said tissue, preferably, wherein the tissue is a soft tissue.

21. A sealant layer obtained by the use of the composition according to claim 20.

22. The sealant layer of claim 21, **characterized by** minimum bond strength of at least 3.5 N/m as measured by T-peel test (ASTM F2256-052015).

23. A composition comprising: (i) a blend of fibrinogen and thrombin, wherein the blend is in the form of a plurality of particles, and (ii) a hydrophobic dispersant comprising mineral oil or soybean oil (iii) one or more components selected from calcium salt and/or lysine, wherein:

   the ratio of said blend to the hydrophobic dispersant ranges from 0.5:1 to 0.9:1 (w/w), respectively;
   the plurality of particles is **characterized by** a median particle size of about 8 to about 60 $\mu$m, and wherein the composition is in the form of a paste at at-least one temperature in the range of 10 °C to 37 °C.

24. A kit comprising:

   a. a container containing the composition of in any one of claims 1 to 18, and 23;
   b. an applicator for applying the composition to a tissue, and optionally
   c. instructions for use.

**Patentansprüche**

1. Zusammensetzung, umfassend: (i) eine Mischung aus Fibrinogen und Thrombin, wobei die Mischung in Form einer Vielzahl von Partikeln vorliegt, und (ii) ein hydrophobes Dispersionsmittel, wobei die Zusammensetzung bei mindestens einer Temperatur im Bereich von 10 °C bis 37 °C in Form einer Paste vorliegt.

2. Zusammensetzung nach Anspruch 1, wobei die Paste ferner Kollagen umfasst.

3. Zusammensetzung nach Anspruch 2, wobei das Kollagen in einer Konzentration von unter 20 Gew.-% vorliegt, wobei das Kollagen wahlweise in einer Konzentration von mindestens etwa 10 Gew.-% vorliegt.

4. Zusammensetzung nach einem der Ansprüche 1 bis 3, wobei die Paste **gekennzeichnet ist durch** eine Fließgrenze im Bereich von etwa 0,1 bis etwa 15 Pa, gemessen bei einer Temperatur von 25 °C und einer Frequenz von 10 Rad/Sek.

5. Zusammensetzung nach einem der Ansprüche 1 bis 4, wobei das Verhältnis der Mischung zum hydrophoben Dispersionsmittel jeweils im Bereich von 0,5 : 1 bis weniger als 0,9 : 1 (Gew./Gew.) liegt.

6. Zusammensetzung nach einem der Ansprüche 1 bis 5, wobei das hydrophobe Dispersionsmittel ausgewählt ist aus der Gruppe, bestehend aus Pflanzenöl, Mineralöl und einer Kombination davon, wobei das hydrophobe Dispersionsmittel vorzugsweise Mineralöl umfasst.

7. Zusammensetzung nach einem der Ansprüche 1 bis 6, wobei das hydrophobe Dispersionsmittel ein Öl umfasst, ausgewählt aus der Gruppe, bestehend aus Sojaöl, Olivenöl, Cholesteryloleat, Maisöl, Triolein, Distelöl, Squalen, Squalan, Dodecan und einer beliebigen Mischung davon.

8. Zusammensetzung nach einem der Ansprüche 1 bis 7, wobei das Fibrinogen in einem Konzentrationsbereich von 60 Gew.-% bis 95 Gew.-% oder 70 Gew.-% bis 90 Gew.-% der Mischung vorliegt, wobei das Fibrinogen vorzugsweise in einem Konzentrationsbereich von 60 Gew.-% bis 85 Gew.-% der Mischung vorliegt und das Thrombin in einem Konzentrationsbereich von 5 Gew.-% bis 10 Gew.-% der Mischung vorliegt.

9. Zusammensetzung nach einem der Ansprüche 1 bis 8, wobei die Mischung weniger als 6 Gew.-% Wasser umfasst.

10. Zusammensetzung nach einem der Ansprüche 1 bis 9, umfassend weniger als 3,5 % Wasser, bezogen auf das Gesamtgewicht.

**11.** Zusammensetzung nach einem der Ansprüche 1 bis 10, wobei die Mischung ferner einen oder mehrere Exzipienten umfasst, ausgewählt aus der Gruppe, bestehend aus: Aminosäuren, Albumin, Sacchariden oder einem beliebigen Derivat oder Gemisch davon.

**12.** Zusammensetzung nach Anspruch 1 bis 11, ferner umfassend ein Calciumsalz und/oder Lysin.

**13.** Zusammensetzung nach einem der Ansprüche 1 bis 12, wobei die Vielzahl der Partikel **gekennzeichnet ist durch** eine mittlere Partikelgröße von bis zu 270 μm, wobei vorzugsweise die Vielzahl der Partikel **gekennzeichnet ist durch** eine mittlere Partikelgröße von etwa 8 bis etwa 60 μm.

**14.** Zusammensetzung nach einem der Ansprüche 1 bis 13, umfassend oxidierte Zellulose (OC) in einer Konzentration von weniger als 12 %, z. B. 5 bis weniger als 12 %, wobei die oxidierte Zellulose (OC) vorzugsweise oxidierte regenerierte Zellulose (ORC) ist.

**15.** Zusammensetzung nach einem der Ansprüche 1 bis 13, die frei von oxidierter Zellulose (OC) ist, wobei die oxidierte Zellulose (OC) vorzugsweise oxidierte regenerierte Zellulose (ORC) ist.

**16.** Zusammensetzung nach einem der Ansprüche 1 bis 15, wobei die Mischung sprühgetrocknet gemischt ist und/oder wobei die Mischung unter hoher Scherung gemischt ist.

**17.** Zusammensetzung nach einem der Ansprüche 1 bis 16, wobei das Thrombin und/oder Fibrinogen aus Schweineplasma stammt.

**18.** Zusammensetzung nach einem der Ansprüche 1 bis 17, die eine hämostatische Zusammensetzung ist.

**19.** Zusammensetzung nach einem der Ansprüche 1 bis 18 zur Verwendung in einem Verfahren zur Behandlung von blutendem Gewebe.

**20.** Zusammensetzung zur Verwendung nach Anspruch 19, wobei die Zusammensetzung auf eine Oberfläche des Gewebes aufgetragen wird, wobei das Gewebe vorzugsweise ein Weichgewebe ist.

**21.** Versiegelungsschicht, erhalten durch die Verwendung der Zusammensetzung nach Anspruch 20.

**22.** Versiegelungsschicht nach Anspruch 21, **gekennzeichnet durch** Mindesthaftfestigkeit von mindestens 3,5 N/m, gemessen mittels T-Schälprüfung (ASTM F2256-052015).

**23.** Zusammensetzung, umfassend: (i) eine Mischung aus Fibrinogen und Thrombin, wobei die Mischung in Form einer Vielzahl von Partikeln vorliegt, und (ii) ein hydrophobes Dispersionsmittel, umfassend Mineralöl oder Sojaöl (iii), eine oder mehrere Komponenten, ausgewählt aus Calciumsalz und/oder Lysin, wobei:

das Verhältnis der Mischung zum hydrophoben Dispersionsmittel jeweils im Bereich von 0,5 : 1 bis 0,9 : 1 (Gew./Gew.) liegt;
die Anzahl der Partikel **gekennzeichnet ist durch** eine mittlere Partikelgröße von etwa 8 bis etwa 60 μm, und wobei die Zusammensetzung bei mindestens einer Temperatur im Bereich von 10 °C bis 37 °C in Form einer Paste vorliegt.

**24.** Kit, umfassend:

a. einen Behälter, enthaltend die Zusammensetzung nach einem der Ansprüche 1 bis 18 und 23;
b. einen Applikator zum Auftragen der Zusammensetzung auf ein Gewebe und wahlweise
c. eine Gebrauchsanweisung.

**Revendications**

**1.** Composition comprenant : (i) un mélange de fibrinogène et de thrombine, dans laquelle le mélange se présente sous la forme d'une pluralité de particules, et (ii) un dispersant hydrophobe, dans laquelle la composition se présente sous la forme d'une pâte à au moins une température dans la plage allant de 10 °C à 37 °C.

**2.** Composition selon la revendication 1, dans laquelle la pâte comprend en outre du collagène.

**3.** Composition selon la revendication 2, dans laquelle le collagène est présent à une concentration inférieure à 20 % en poids, éventuellement dans laquelle le collagène est présent à une concentration d'au moins environ 10 % en poids.

**4.** Composition selon l'une quelconque des revendications 1 à 3, dans laquelle la pâte est **caractérisée par** une limite apparente d'élasticité allant d'environ 0,1 à environ 15 Pa, telle que mesurée à une température de 25 °C et à une fréquence de 10 rad/sec.

**5.** Composition selon l'une quelconque des revendications 1 à 4, dans laquelle le rapport dudit mélange au dispersant hydrophobe va de 0,5:1 à moins de 0,9:1 (p/p), respectivement.

**6.** Composition selon l'une quelconque des revendications 1 à 5, dans laquelle le dispersant hydrophobe est choisi dans le groupe constitué d'huile végétale, huile minérale et une combinaison de celles-ci, de préférence, dans laquelle le dispersant hydrophobe comprend de l'huile minérale.

**7.** Composition selon l'une quelconque des revendications 1 à 6, dans laquelle le dispersant hydrophobe comprend une huile choisie dans le groupe constitué d'huile de soja, huile d'olive, oléate de cholestéryle, huile de maïs, trioléine, huile de carthame, squalène, squalane, dodécane et un mélange quelconque de ceux-ci.

**8.** Composition selon l'une quelconque des revendications 1 à 7, dans laquelle le fibrinogène est présent à une concentration allant de 60 % à 95 %, ou de 70 % à 90 %, en poids du mélange, de préférence, dans laquelle le fibrinogène est présent à une concentration allant de 60 % à 85 %, en poids du mélange, et la thrombine est présente à une concentration allant de 5 % à 10 %, en poids du mélange.

**9.** Composition selon l'une quelconque des revendications 1 à 8, dans laquelle le mélange comprend moins de 6 % d'eau, en poids.

**10.** Composition selon l'une quelconque des revendications 1 à 9, comprenant moins de 3,5 % d'eau, en poids total.

**11.** Composition selon l'une quelconque des revendications 1 à 10, dans laquelle le mélange comprend en outre un ou plusieurs excipients choisis dans le groupe constitué de : acides aminés, albumine, saccharides, ou un dérivé ou mélange quelconque de ceux-ci.

**12.** Composition selon les revendications 1 à 11, comprenant en outre un sel de calcium et/ou de la lysine.

**13.** Composition selon l'une quelconque des revendications 1 à 12, dans laquelle la pluralité de particules est **caractérisée par** une taille médiane des particules allant jusqu'à 270 μm, de préférence, dans laquelle la pluralité de particules est **caractérisée par** une taille médiane des particules allant d'environ 8 à environ 60 μm.

**14.** Composition selon l'une quelconque des revendications 1 à 13, comprenant de la cellulose oxydée (OC) à une concentration inférieure à 12 %, par exemple allant de 5 à moins de 12 %, de préférence, dans laquelle la cellulose oxydée (OC) est de la cellulose régénérée oxydée (ORC).

**15.** Composition selon l'une quelconque des revendications 1 à 13, dépourvue de cellulose oxydée (OC), de préférence, dans laquelle la cellulose oxydée (OC) est de la cellulose régénérée oxydée (ORC).

**16.** Composition selon l'une quelconque des revendications 1 à 15, dans laquelle le mélange est mélangé avec séchage par pulvérisation, et/ou dans laquelle le mélange est mélangé à haut cisaillement.

**17.** Composition selon l'une quelconque des revendications 1 à 16, dans laquelle la thrombine et/ou le fibrinogène proviennent de plasma porcin.

**18.** Composition selon l'une quelconque des revendications 1 à 17, laquelle est une composition hémostatique.

**19.** Composition selon l'une quelconque des revendications 1 à 18, pour une utilisation dans un procédé pour le traitement de tissu qui saigne.

**20.** Composition pour une utilisation selon la revendication 19, dans laquelle la composition est appliquée sur une surface dudit tissu, de préférence, dans laquelle le tissu est un tissu mou.

**21.** Couche d'étanchéité obtenue par l'utilisation de la composition selon la revendication 20.

**22.** Couche d'étanchéité selon la revendication 21, **caractérisée par** une résistance d'adhésion minimale d'au moins 3,5 N/m telle que mesurée par l'essai de pelage en T (norme ASTM F2256-052015).

**23.** Composition comprenant : (i) un mélange de fibrinogène et de thrombine, dans laquelle le mélange se présente sous la forme d'une pluralité de particules, et (ii) un dispersant hydrophobe comprenant de l'huile minérale ou de l'huile de soja (iii) un ou plusieurs composants choisis parmi sel de calcium et/ou lysine, dans laquelle :

le rapport dudit mélange au dispersant hydrophobe va de 0,5:1 à 0,9:1 (p/p), respectivement ;
la pluralité de particules est **caractérisée par** une taille médiane des particules allant d'environ 8 à environ 60 $\mu$m, et dans laquelle la composition se présente sous la forme d'une pâte à au moins une température dans la plage allant de 10 °C à 37 °C.

**24.** Kit comprenant :

a. un récipient contenant la composition selon l'une quelconque des revendications 1 à 18 et 23 ;
b. un applicateur pour l'application de la composition sur un tissu, et éventuellement
c. des instructions pour l'utilisation.

Fig. 1A

Fig. 1B

Fig. 1C

Fig. 1D

Fig. 2

# Main Effects Plot for gelation time /min
## Fitted Means

Fig. 3A

Fig. 3B

**Interval Plot of HSMP250/MO, HSMP/PEG400, ...**
95% CI for the Mean

Fig. 4A

**Tukey Simultaneous 95% CIs**
Difference of Means for HSMP250/MO, HSMP/PEG400, ...

Fig. 4B

Fig. 5

**Main Effects Plot for T-peel N/m**
Fitted Means

Fig. 6A

Fig. 6B

## Remnant in syringe

Fig. 7

Fig. 8

Fig. 9

| * | ** | *** | **** | ***** |

Fig. 10A

| Sample no. | Bleeding site | Apply formulation | After 1min tamponade |
|:---:|:---:|:---:|:---:|
| 1 | | | |
| 2 | | | |
| 3 | | | |
| 4 | | | |

Fig. 10B

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 4965203 A
- EP 1140235 A
- US 9717821 B
- US 7109163 B
- WO 2013082073 A
- WO 2013079604 A

- WO 9816250 A
- WO 2019160717 A
- WO 2015086028 A
- WO 2018033835 A
- WO 2013079605 A
- WO 2007110694 A

### Non-patent literature cited in the description

- *Arterioscler Thromb. Vasc. Biol.*, 1998, 1948-1957
- *CHEMICAL ABSTRACTS*, 8020-83-5

- Remington's Pharmaceutical Sciences. Mack Publishing Company, 1990, 788-1323
- **VU et al.** *J. Viet. Env.*, 2016, vol. 8 (1), 21-25
- *CHEMICAL ABSTRACTS*, 8001-25-0